# EUROPEAN PATENT APPLICATION

(11) **EP 4 775 139 A1**
(43) Date of publication of application: **15.07.2026**
(21) Application number: 25781611.6
(22) Date of filing: 27.03.2025
(51) Int. Cl.: A61B 5/0205, A61B 5/1455, A61B 5/332, A61B 5/00, A61B 5/318, A61B 5/28

(54) **HEALTH MONITORING MODULE AND ELECTRONIC DEVICE**

(30) Priority: 01.04.2024 CN 202410390337
(71) Applicant: HUAWEI TECHNOLOGIES CO., LTD., Shenzhen 518129 (CN)
(72) Inventor: LIU, Zhuang, Shenzhen, Guangdong 518129 (CN); ZOU, Lin, Shenzhen, Guangdong 518129 (CN); JIN, Junye, Shenzhen, Guangdong 518129 (CN); YANG, Wenjian, Shenzhen, Guangdong 518129 (CN); ZHAO, Menglong, Shenzhen, Guangdong 518129 (CN); ZHANG, Ze, Shenzhen, Guangdong 518129 (CN)
(74) Representative: Gill Jennings & Every LLP
(86) International application number: PCT/CN2025/085415
(87) International publication number: WO 2025/209314

(57) **Abstract**

This application provides a health monitoring module and an electronic device. The health monitoring module includes an enclosure, an optical heart rate module, a circuit board, and a light-transmitting member. The enclosure is fastened to a side of the circuit board, and the enclosure and the circuit board jointly enclose accommodation space. The enclosure includes a conductive portion, the conductive portion is made of a conductive material, a part of the conductive portion protrudes from a top face of the enclosure, and another part is electrically connected to the circuit board. The optical heart rate module is located in the accommodation space, the optical heart rate module is fastened to the circuit board and is electrically connected to the circuit board, the optical heart rate module and a light-transmitting hole are disposed oppositely, and the optical heart rate module emits and receives light beams through the light-transmitting hole and the light-transmitting member. The optical heart rate module may be configured to collect a PPG signal of a user. The conductive portion may be configured to collect an electrical signal of the user. After the electrical signal is processed, an ECG signal of the user may be obtained. A component for measuring the ECG signal and the PPG signal is integrated into one module, which helps achieve multifunctionality of the module in a small size.

## Description

This application claims priority to Chinese Patent Application No. 202410390337.1, filed with the China National Intellectual Property Administration on April 1, 2024 and entitled "HEALTH MONITORING MODULE AND ELECTRONIC DEVICE", which is incorporated herein by reference in its entirety.

### TECHNICAL FIELD

This application relates to the field of electronic technologies, and in particular, to a health monitoring module and an electronic device.

### BACKGROUND

In today's society, people have increasingly high requirements for electronic device functions, and users hope that more health monitoring functions can be integrated into small-sized electronic devices. For example, the user expects that the electronic device can monitor a plurality of indicators such as blood oxygen, an electrocardiogram, a heart rate, and blood pressure, and perform analysis based on the plurality of indicators, to form a micro-physical examination mode or a pressure monitoring mode. This helps the user monitor physical health. Therefore, how to achieve multifunctionality of the electronic device while ensuring miniaturization and portability of the electronic device becomes a development trend of the industry.

### SUMMARY

This application provides a health monitoring module and an electronic device.

According to a first aspect, an embodiment of this application provides a health monitoring module. The health monitoring module includes an enclosure, an optical heart rate module, a circuit board, and a light-transmitting member. The enclosure is fastened to a side of the circuit board, the enclosure and the circuit board jointly enclose accommodation space, a light-transmitting hole is disposed in the enclosure, and the light-transmitting hole communicates the accommodation space with the outside. The enclosure includes a conductive portion, the conductive portion is made of a conductive material, a part of the conductive portion protrudes from a top face of the enclosure, and another part is electrically connected to the circuit board. The light-transmitting member is fastened to the enclosure and at least partially filled in the light-transmitting hole. The optical heart rate module is located in the accommodation space, the optical heart rate module is fastened to the circuit board and is electrically connected to the circuit board, the optical heart rate module and the light-transmitting hole are disposed oppositely, and the optical heart rate module emits and receives light beams through the light-transmitting hole and the light-transmitting member.

It may be understood that the enclosure is fastened to the side of the circuit board, and the enclosure and the circuit board jointly enclose the accommodation space. The optical heart rate module is located in the accommodation space. The enclosure can protect the optical heart rate module.

The light-transmitting hole is disposed in the enclosure, and the light-transmitting hole communicates the accommodation space with the outside. The light-transmitting member is fastened to the enclosure and at least partially filled in the light-transmitting hole. The light-transmitting member is configured to seal the light-transmitting hole, to prevent external impurities such as dust from interfering with operation of the optical heart rate module by entering the accommodation space through the light-transmitting hole.

The optical heart rate module may be configured to collect a heart rate signal of a user, for example, a photoplethysmogram (photoplethysmogram, PPG) signal. When a finger of the user presses the enclosure, the finger of the user may cover the light-transmitting hole, and the light beams emitted by the optical heart rate module may irradiate the finger of the user through the light-transmitting hole. The light beams may be reflected or scattered by blood vessels of the finger of the user, and return to the accommodation space through the light-transmitting hole. The light beams are received by the optical heart rate module. An electrical signal is formed through optical-to-electrical conversion, and is transmitted to the circuit board.

The light beams reflected or scattered by the blood vessels of the finger of the user may reflect blood flow information of the user. Blood flow usually refers to flow of blood in blood vessels of a body. The blood flow is a basic parameter of a blood circulatory system and reflects flow of the blood through arteries, capillaries, and veins under action of pumping of the blood by the heart. The blood flow may be represented based on a plurality of parameters, including but not limited to a speed, a flow rate, a direction, stability (whether the blood flows stably in the circulatory system), resistance (the blood flow is affected by resistance of blood vessel walls), pulsatility (fluctuation of the blood flow in a cardiac pulsation process), distribution (distribution of the blood flow in the body), and the like. The blood flow information is measured and analyzed to evaluate a function of a cardiovascular system, diagnose cardiovascular diseases, and develop effective treatment plans.

The blood flow information and other physiological features such as a heart rate, blood oxygen, and breathing are associated with each other and affect each other. In physiological feature monitoring, comprehensive consideration of the information helps understand physiological and health statuses of the body more accurately.

A part of the conductive portion protrudes from a top face of the enclosure. When the user needs to measure an electrocardiogram, the finger may be in contact with the top face of the enclosure, and is in an electrical connection to the conductive portion, so that the enclosure can collect the electrical signal of the user. An electrocardiogram (Electrocardiogram, ECG) waveform of the user can be obtained through processing of the electrical signal. The electrocardiogram waveform can reflect a cardiac function status of the user.

The PPG signal may be further combined with the foregoing ECG waveform, and more accurate blood pressure of the user and other biological feature information related to the human cardiovascular system are obtained according to an algorithm. For example, the health monitoring module may collect both the ECG waveform and the PPG signal of the user, calculate a time difference PPT between the ECG waveform and the PPG signal according to an algorithm, and obtain blood pressure data of the user through conversion according to a formula.

The enclosure may be further configured to sense or detect touch or pressing action of the user, so that the health monitoring module has a button function. For example, when the user touches or presses the conductive portion, the conductive portion may collect the electrical signal of the user. It is determined, based on whether the conductive portion collects the electrical signal of the user, whether the user touches or presses the conductive portion.

The enclosure of the health monitoring module in this application may be configured to: collect the electrical signal of the user, and obtain an ECG signal. The optical heart rate module may be configured to obtain the PPG signal of the user. After the ECG signal and the PPG signal are processed according to corresponding algorithms, a plurality of physiological indicators of the user such as the electrocardiogram, the heart rate, and the blood pressure can be obtained. In a conventional technical solution, a PPG collection apparatus and an ECG collection apparatus are separately disposed. Two fingers of the user need to separately be in contact with the PPG collection apparatus and the ECG collection apparatus, to complete collection of the ECG signal and the PPG signal. When the health monitoring module in this application collects the ECG signal and the PPG signal of the user, the user only needs to place one finger on the top face of the enclosure, so that an operation is more convenient.

The health monitoring module integrates the button function and a health monitoring function. In comparison with a solution in which a button function module and a health monitoring function module are separately disposed, the health monitoring module in this application achieves multifunctionality and has a small size.

In addition, in this application, a blood pressure signal of the user is measured based on the combination of the ECG waveform and the PPG signal. In comparison with a conventional product such as a sphygmomanometer, no mechanisms such as an air pump and a bladder need to be disposed, and a size is smaller. When the health monitoring module in this application measures the blood pressure, the user only needs to place the finger on the enclosure to complete blood pressure monitoring by one tap. A monitoring step is simple, facilitating the user.

In an implementation, the health monitoring module further includes a pressure sensing circuit and a pressure sensing assembly, the pressure sensing assembly is fastened to a side that is of the circuit board and that faces away from the enclosure, and the pressure sensing circuit is fastened to the pressure sensing assembly. When the enclosure is subject to pressure, and the pressure sensing assembly is deformed, the pressure sensing circuit is configured to detect the deformation of the pressure sensing assembly.

It may be understood that, when the user measures the ECG signal and the PPG signal, the user places the finger on the enclosure, and the pressure sensing assembly may be deformed to different degrees based on an action force between the finger of the user and the enclosure. The pressure sensing circuit may be configured to detect the deformation of the pressure sensing assembly. In this way, a deformation degree, of the pressure sensing assembly, detected by the pressure sensing circuit may be used to obtain a magnitude of a force at which the finger of the user presses the enclosure.

When the finger of the user is placed at an incorrect position on the enclosure, or when the finger is placed at a position that is offset from an ideal monitoring position, the pressure sensing assembly is subject to a small force. A pressure sensing chip may determine, based on a force exerted on the pressure sensing assembly, whether the finger of the user is placed in place. In operating processes of the pressure sensing circuit and the pressure sensing assembly, a first threshold may be set. The first threshold is a minimum force exerted on the pressure sensing assembly when the finger of the user is placed in place. When the pressure sensing chip detects that a real-time exerted force detected by the pressure sensing assembly is greater than or equal to the first threshold, the pressure sensing chip may control the health monitoring module to start to collect both the ECG signal and the PPG signal of the user, calculate a time difference between the ECG signal and the PPG signal, that is, pulse transit time (Pulse Transit Time, PTT), and finally obtain the blood pressure data of the user through conversion according to a series of formulas.

When the action force between the finger of the user and the enclosure is small, depths of tissue penetrated, beneath the skin of the user, by light beams emitted by a light emitter are limited, and the blood vessels of the user are at specific depths beneath the skin surface of the user. Generally, a larger value of the force of the user exerting on the enclosure indicates better fitting effect between the finger and a knob, and greater depths to which the light beams emitted by the light emitter penetrate the skin of the user. Therefore, when the health monitoring module is configured to measure the PPG signal, a second threshold may be set. When the action force between the finger of the user and the enclosure is greater than or equal to the second threshold, the light beams emitted by the light emitter penetrate the skin of the user to great depths, so that most of the light beams can irradiate positions of the blood vessels beneath the skin of the user. When the pressure sensing chip detects that the real-time exerted force detected by the pressure sensing assembly is greater than or equal to the second threshold, the pressure sensing chip controls the health monitoring module to start to collect the PPG signal of the user. In this way, accuracy of the collected PPG signal is good.

In addition, when the real-time exerted force detected by the pressure sensing assembly is greater than or equal to the second threshold, the pressure sensing chip controls the health monitoring module to start to collect the ECG signal of the user, so that the action force between the user and the enclosure is within a large range, and reliability of the electrical connection between the enclosure and the user is good. In this way, accuracy of the collected ECG signal is good.

In addition, the pressure sensing circuit and the pressure sensing assembly may be configured to measure a water depth. When the user is underwater, water pressure can also cause the pressure sensing assembly to deform, so that the pressure sensing circuit can detect a deformation degree of the pressure sensing assembly. The pressure sensing chip can detect a value of the water pressure based on an electrical signal of the pressure sensing circuit. The water pressure is proportional to the water depth. Real-time underwater depth information of the user may be obtained through calculation according to a formula, so that the health monitoring module can have an underwater depth detection function.

In an implementation, the pressure sensing assembly includes a stem, and a part of region of a first surface of the stem is fastened to the side that is of the circuit board and that faces away from the enclosure. The pressure sensing circuit is disposed in another part of region of the first surface of the stem, and the pressure sensing circuit and the circuit board are spaced apart. When the enclosure is subject to the pressure, and the stem is deformed, the pressure sensing circuit is configured to detect the deformation of the stem.

It may be understood that the pressure sensing circuit is mounted on the first surface of the stem rather than another position of the stem. The part of region of the first surface of the stem may be fastened to the side that is of the circuit board and that faces away from the enclosure. A distance between the pressure sensing circuit and the circuit board may be short. When the pressure sensing circuit is electrically connected to the circuit board, a length of an electrical connection structure between the pressure sensing circuit and the circuit board is short, and a size of the electrical connection structure is small. This facilitates miniaturization of the health monitoring module.

In addition, the pressure sensing circuit and the circuit board are spaced apart. Space between the pressure sensing circuit and the circuit board may be used to provide space for the deformation of the stem, to prevent the stem from pressing another nearby component when the stem is deformed.

In an implementation, a groove is disposed on the first surface of the stem, and the pressure sensing circuit is disposed in the groove.

It may be understood that the first surface of the stem may be recessed towards a side away from the circuit board to form the groove, and a wall face of the groove is a part of the first surface of the stem. A depth of the groove may be set to be slightly greater than thickness of the pressure sensing circuit, and the pressure sensing circuit is fastened to a bottom face of the groove. The pressure sensing circuit and the circuit board can be spaced.

In an implementation, the stem includes a first part and a second part, the first part is plate-shaped, the second part is rod-shaped, the second part is located on a side that is of the first part and that faces away from the circuit board, and an end of the second part is connected to a middle of the first part. The first surface of the stem is a surface of a side that is of the first part and that faces away from the second part, and when the enclosure is subject to the pressure, and the first part is deformed, the pressure sensing circuit is configured to detect the deformation of the first part.

It may be understood that the stem is set to a "T" structure. Two ends of the first part may be fastened to the circuit board. Connection positions between the first surface and the circuit board may include a first position P and a second position P (as shown in the figure). The groove may be disposed between the first position P and the second position P. When the enclosure is subject to the pressure, the two ends of the first part are subject to two forces F, and the middle of the first part is subject to a force F, so that the deformation of the first part can be large, and a resistance change of the pressure sensing circuit is large. This facilitates detection of the pressure applied by the user on the enclosure. In addition, when the user presses the enclosure, the enclosure is not easy to tilt towards one side.

In an implementation, the health monitoring module further includes a first waterproof member, and the first waterproof member is connected to the first part and covers the pressure sensing circuit; and/or
the health monitoring module further includes a second waterproof member, and the second waterproof member is sleeved on the second part.

It may be understood that, the first waterproof member may be configured to implement waterproofing and dustproofing for the pressure sensing circuit, to prevent water vapor or dust from interfering with operation of the pressure sensing circuit through a gap between the circuit board and the stem.

When the health monitoring module is fastened to a middle frame of the electronic device, a fastening hole may be disposed on the middle frame, and a part of the second part is located in the fastening hole. In this case, the second waterproof member may abut against the second part and a wall face of the fastening hole. It may be understood that, a probability that operation of a component (for example, a mainboard) in internal space of the electronic device is interfered with by the external water vapor or dust entering the internal space of the electronic device through a gap between the second part and the fastening hole can be reduced by disposing the second waterproof member.

In an implementation, the pressure sensing assembly includes a stem and a support steel sheet, and a first surface of the stem is fastened to the side that is of the circuit board and that faces away from the enclosure. The support steel sheet is connected to a second surface of the stem, the first surface of the stem and the second surface of the stem are disposed back to back, and the pressure sensing circuit is fastened to a side that is of the support steel sheet and that is away from the stem. When the enclosure is subject to the pressure, and the support steel sheet is deformed, the pressure sensing circuit is configured to detect the deformation of the support steel sheet.

It may be understood that the pressure sensing circuit and the circuit board are spaced apart. When the pressure sensing circuit is faulty, the pressure sensing circuit may be separately repaired, and the optical heart rate module is not affected, so that the repair is more convenient.

In an implementation, the pressure sensing assembly further includes an elastic member, the elastic member abuts against the stem and the support steel sheet, and the elastic member is elastic.

It may be understood that, by disposing the elastic member, structural tolerances of the stem and the support steel sheet can be accommodated, and assembly tolerances between the stem and the support steel sheet can be accommodated, to avoid that an excessively large abutting force between the stem and the support steel sheet causes large deformation of the support steel sheet, leading to reduction in pressure sensing detection accuracy or a pressure sensing detection failure. In addition, when the health monitoring module is used as a button, and the user presses the health monitoring module, the elastic member may be used for cushioning, and the elastic member may further improve a tactile feeling for use.

In an implementation, the pressure sensing assembly includes a stem and a support steel sheet, a part of region of a first surface of the stem is fastened to the side that is of the circuit board and that faces away from the enclosure, the support steel sheet is connected to a second surface of the stem, and the second surface of the stem and the first surface of the stem are disposed back to back.

The pressure sensing circuit includes a first pressure sensing circuit and a second pressure sensing circuit, the first pressure sensing circuit is disposed in another part of region of the first surface of the stem, the first pressure sensing circuit and the circuit board are spaced apart, and the second pressure sensing circuit is fastened to a side that is of the support steel sheet and that is away from the stem.

When the enclosure is subject to the pressure, the stem is deformed, and the support steel sheet is deformed, the first pressure sensing circuit is configured to detect the deformation of the stem, and the second pressure sensing circuit is configured to detect the deformation of the support steel sheet.

It may be understood that the two pressure sensing circuits, that is, the first pressure sensing circuit and the second pressure sensing circuit, are disposed, and the first pressure sensing circuit and the second pressure sensing circuit are spaced apart. In comparison with an implementation in which only one pressure sensing circuit is disposed, in this implementation, a plurality of pressure sensing circuits are disposed, so that pressure sensing detection accuracy is higher. This helps improve collection of the ECG signal and the PPG signal. In addition, when one of the pressure sensing circuits fails, another pressure sensing circuit may still operate normally, and a pressure sensing detection function of the health monitoring module is reliable.

In an implementation, the enclosure and the light-transmitting member are an integrally formed mechanical part; and/or
the light-transmitting member is in contact with and fastened to the enclosure, the optical heart rate module, and the circuit board.

It may be understood that connection strength between the enclosure and the light-transmitting member is good. For example, when the light-transmitting member is formed through curing of glue, the transparent glue may be injected into the enclosure via a jig. After the glue is cured, the light-transmitting member is directly fastened to the enclosure to form the mechanical part. In this way, a connection structure does not need to be additionally disposed between the light-transmitting member and the enclosure for the health monitoring module. This simplifies a technical process and helps reduce costs of the health monitoring module. In addition, no additional connection structure needs to be disposed between the enclosure and the light-transmitting member, and there is no visual segment gap between the enclosure and the light-transmitting member, providing good appearance.

The light-transmitting member is in contact with and fastened to the enclosure, the optical heart rate module, and the circuit board. The light-transmitting member can implement a waterproofing function for the optical heart rate module.

In an implementation, a first through hole and a second through hole that are spaced apart are disposed on the circuit board, both the first through hole and the second through hole communicate the accommodation space with the outside, and the light-transmitting member is filled in the accommodation space, the first through hole, and the second through hole.

The optical heart rate module includes a light emitter and a light receiver, and the light emitter and the light receiver are spaced apart on the circuit board. The light emitter is located between the first through hole and the second through hole, or the light receiver is located between the first through hole and the second through hole.

It may be understood that, when the light-transmitting member may be formed by using a potting process, either the first through hole or the second through hole may be used as a glue filling hole, and the other may be used as a glue outlet hole.

In comparison with a solution in which both the light emitter and the light receiver are located between the first through hole and the second through hole, the light emitter is located between the first through hole and the second through hole, or the light receiver is located between the first through hole and the second through hole. Large tracing space may be reserved on a right side of the light emitter or on a left side of the light receiver. The tracing space may be used to arrange a signal transmission line, and transmit signals of the conductive portion and the light receiver.

In an implementation, the optical heart rate module includes the light emitter and the light receiver, the light emitter and the light receiver are spaced apart and fastened to the circuit board, there are two light-transmitting holes, the two light-transmitting holes are spaced apart, and the light emitter and the light receiver are respectively disposed in correspondence with the two light-transmitting holes.

The enclosure includes a frame portion and a light-shielding portion, the frame portion is connected to the circuit board, the frame portion and the circuit board enclose the accommodation space, the light-transmitting holes are disposed in the frame portion, the light-shielding portion is connected to the frame portion and located in the accommodation space, the light-shielding portion is at least partially located between the light emitter and the light receiver, and the light-shielding portion is made of an opaque material.

It may be understood that, the light-shielding portion may block the light beams emitted by the light emitter, and reduce a probability that the light beams emitted by the light emitter are directly propagated to a position of the light receiver in the accommodation space, so that the light receiver receives, as much as possible, light beams that are all reflected by the user, thereby improving measurement accuracy of the heart rate signal.

In an implementation, the optical heart rate module and the enclosure are spaced apart.

It may be understood that the optical heart rate module and the enclosure are spaced apart, and a gap between the enclosure and the optical heart rate module may be used to dispose a waterproof structure, to perform waterproof and dustproof protection on the optical heart rate module. In addition, the gap between the enclosure and the optical heart rate module may be further used to accommodate an assembly error, to avoid damage to either of the enclosure or the optical heart rate module caused by abutting or collision of the enclosure and the optical heart rate module in an assembly process.

In an implementation, the light-transmitting member is further filled in the accommodation space and wraps the optical heart rate module.

It may be understood that the light-transmitting member can implement waterproof, dustproof, and protection functions for the optical heart rate module.

In an implementation, the health monitoring module further includes a conductive member, the conductive member is located in the accommodation space and is spaced apart from the optical heart rate module, the conductive member abuts against the conductive portion and the circuit board and is electrically connected between the conductive portion and the circuit board, and the conductive member is elastic.

It may be understood that the conductive member may be electrically connected between the conductive portion and the circuit board, to transmit the electrical signal collected by the conductive portion to the circuit board. The conductive member may be elastic. The conductive member may abut against the conductive portion of the enclosure and the circuit board, and is electrically connected to the conductive portion and the circuit board. In comparison with a non-elastic conductive structure such as a metal trace or a flexible printed circuit board, the conductive member is elastic, the conductive member abuts against the conductive portion of the enclosure and the circuit board, and the conductive member does not easily shift, so that reliability of the electrical connection between the conductive member and the conductive portion, and reliability of the electrical connection between the conductive member and the circuit board can be improved.

In an implementation, a hole is disposed on the conductive member; and/or
a part of region of a first surface of the conductive member is connected to the circuit board, another part of region of the first surface of the conductive member is recessed in a direction away from the circuit board and is spaced apart from the circuit board.

It may be understood that the hole may be disposed on the conductive member. When the conductive member abuts against the conductive portion of the enclosure and the circuit board, the hole may provide deformation space.

First space is formed between the another part of region of the first surface and the circuit board. When the conductive member abuts against the conductive portion of the enclosure and the circuit board, the first space may provide deformation space.

In an implementation, the health monitoring module further includes a conducting layer disposed on the top face of the enclosure, the conducting layer is electrically connected to the conductive portion, and material hardness of the conducting layer is greater than material hardness of the conductive portion.

It may be understood that, the conducting layer may be configured to prevent the collection of the electrical signal of the user from being affected by adverse factors such as a scratch, corrosion, and collision on the top face of the enclosure.

In an implementation, the health monitoring module further includes a flexible printed circuit board, the flexible printed circuit board is connected to the circuit board and is electrically connected to the circuit board, and the flexible printed circuit board is located outside the accommodation space.

It may be understood that the flexible printed circuit board may be configured to transmit an electrical signal of the circuit board. The flexible printed circuit board has good bending performance, and can be applicable to a large quantity of electrical connection scenarios.

According to a second aspect, an embodiment of this application provides an electronic device. The electronic device includes a housing and the health monitoring module. The health monitoring module is mounted on the housing.

It may be understood that, when the health monitoring module is mounted on the electronic device, miniaturization of the electronic device is facilitated. The enclosure and an optical heart rate module may be stacked in a thickness direction (a Z-axis direction) of the health monitoring module. The health monitoring module may occupy a small surface of the electronic device. This helps save stacking space of the entire device. More other functional components may be disposed on a surface of the electronic device, to meet diversified requirements of a user.

In an implementation, the electronic device further includes an ECG chip, and the ECG chip is located inside the housing and is electrically connected to the conductive portion of the enclosure of the health monitoring module.

It may be understood that the ECG chip may be configured to process an electrical signal obtained by the enclosure, to obtain an electrocardiogram waveform of the user.

In an implementation, the electronic device further includes a screen, the housing includes a middle frame and a rear cover, the middle frame is connected between the screen and the rear cover, and the health monitoring module is mounted on the middle frame and protrudes from an outer surface of the middle frame.

It may be understood that the outer surface of the middle frame is a surface that is of the middle frame and that is away from internal space of the electronic device, that is, a side face of the electronic device. The health monitoring module is mounted on the side face of the electronic device. This helps the user touch or press the health monitoring module. In comparison with a solution in which the health monitoring module is mounted on the screen, mounting the health monitoring module on the side face of the electronic device does not need to occupy a display region area of the screen. This helps increase a display area of the electronic device.

In an implementation, the electronic device is a watch or a band, and the health monitoring module is a button of the watch or the band.

It may be understood that, a health monitoring function and a button function of the electronic device are integrated into the health monitoring module, so that space in which the button is additionally disposed can be saved for the electronic device, thereby facilitating miniaturization of the electronic device.

In an implementation, the electronic device further includes a motor, the motor is mounted on the housing, and the motor vibrates based on data monitored by the health monitoring module.

It may be understood that, when the health monitoring module is used as a touch-sensitive button of the electronic device, the motor may vibrate to prompt the user whether an operation on the button is effective. The motor generates vibration, to implement a function similar to the touch-sensitive button.

### BRIEF DESCRIPTION OF DRAWINGS

To describe the technical solutions in embodiments of this application, the following describes the accompanying drawings used in embodiments of this application.
FIG. 1 is a diagram of an electronic device in an implementation according to an embodiment of this application;
FIG. 2 is a partial sectional view of the electronic device shown in FIG. 1 along a section line A-A in an implementation;
FIG. 3 is a diagram of a structure of a health monitoring module shown in FIG. 1 in an implementation;
FIG. 4 is an exploded diagram of the health monitoring module shown in FIG. 3 in an implementation;
FIG. 5 is a partial sectional view of the electronic device shown in FIG. 1 along a section line A-A in another implementation;
FIG. 6 is an enlarged diagram of a structure shown in FIG. 5 at B in an implementation;
FIG. 7 is a diagram of a partial structure of a structure shown in FIG. 5 in an implementation;
FIG. 8 is a diagram of a structure of a conductive member shown in FIG. 6 in an implementation;
FIG. 9 is a partial sectional view of the electronic device shown in FIG. 1 along a section line C-C in an implementation;
FIG. 10 is a partial sectional view of the electronic device shown in FIG. 1 along a section line A-A in still another implementation;
FIG. 11 is a partial sectional view of the electronic device shown in FIG. 1 along a section line A-A in yet another implementation;
FIG. 12 is a diagram of a structure of a health monitoring module shown in FIG. 1 in another implementation;
FIG. 13 is an exploded diagram of the health monitoring module shown in FIG. 12 in an implementation;
FIG. 14 is a partial sectional view of the electronic device shown in FIG. 1 along a section line A-A in yet another implementation;
FIG. 15 is a partial sectional view of the electronic device shown in FIG. 1 along a section line C-C in another implementation;
FIG. 16 is a diagram of a structure of a health monitoring module shown in FIG. 1 in another implementation;
FIG. 17 is an exploded diagram of the health monitoring module shown in FIG. 16 in an implementation;
FIG. 18 is a partial sectional view of the electronic device shown in FIG. 1 along a section line A-A in yet another implementation; and
FIG. 19 is a partial sectional view of the electronic device shown in FIG. 1 along a section line C-C in still another implementation.

### DESCRIPTION OF EMBODIMENTS

The following describes embodiments of this application with reference to the accompanying drawings in embodiments of this application. In this specification, embodiments described with reference to the accompanying drawings are examples, and are intended to explain the present invention, but cannot be understood as a limitation on the present invention.

In the descriptions of embodiments of this application, it should be noted that terms "mounting" and "connection" should be understood in a broad sense unless there is a clear stipulation and limitation. For example, "connection" may be a detachable connection, a non-detachable connection, a direct connection, or an indirect connection through an intermediate medium. It should be understood that, in this application, an "electrical connection" may be understood as physical contact and electrical conduction of elements. It may also be understood as a form in which different elements in a line structure are connected through physical lines that can transmit an electrical signal, such as a printed circuit board (printed circuit board, PCB) copper foil or a conducting wire. Both "connected" and "connection" may refer to a mechanical connection relationship or a physical connection relationship. For example, A is connected to B or a connection between A and B may mean that there is a fastening component (for example, a screw, a bolt, or a nail) between A and B, or A and B are in contact with each other and A and B are difficult to be separated.

In addition, "fastening" in this specification should also be understood in a broad sense. For example, "fastening" may be direct fastening or indirect fastening through an intermediate medium. The term "fastening" means that two objects are connected to each other and a relative position relationship remains unchanged after the connection. The orientation terms mentioned in embodiments of this application, for example, "upper" and "lower", are merely directions with reference to the accompanying drawings. Therefore, the orientation terms are used to better and more clearly describe and understand embodiments of this application, rather than indicating or implying that an indicated apparatus or element needs to have a specific orientation or be constructed and operated in the specific orientation, and therefore cannot be construed as a limitation on embodiments of this application. "A plurality of" means to two or more than two.

The terms "first", "second", "third", and "fourth" in embodiments of this application are merely intended for a purpose of description, and shall not be understood as an indication or implication of relative importance or implicit indication of a quantity of indicated technical features. Therefore, a feature defined by "first", "second", "third", or "fourth" may explicitly or implicitly include one or more features.

In the descriptions of embodiments of this application, unless otherwise specified, "and/or" is merely an association relationship for describing an associated object, and indicates that three relationships may exist. For example, A and/or B may represent the following three cases: Only A exists alone, both A and B exist, and only B exists.

It may be understood that the specific embodiments described herein are merely used to explain a related application, but are not intended to limit this application. In addition, it should be further noted that, for ease of description, only a part related to this application is shown in the accompanying drawings.

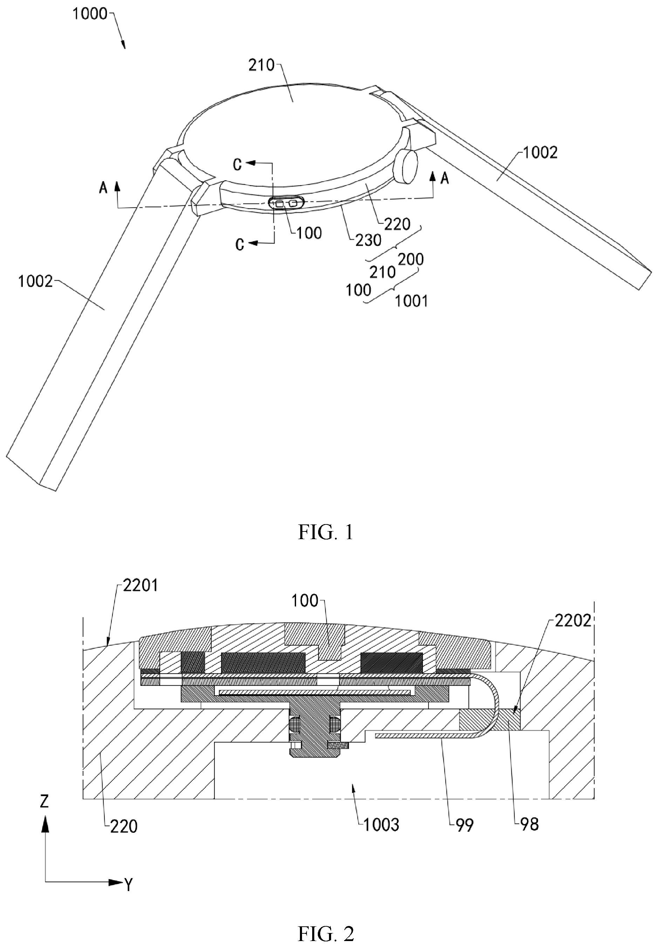

FIG. 1 is a diagram of an electronic device 1000 in an implementation according to an embodiment of this application.

The electronic device 1000 may include but is not limited to a wearable device such as a smartwatch, a sports watch, a band, augmented reality (augmented reality, AR) glasses, virtual reality (virtual reality, VR) glasses, or a headset. Alternatively, the electronic device 1000 may be a terminal product such as a mobile phone, a tablet computer, or a home device. An example in which the electronic device 1000 shown in FIG. 1 is a smartwatch is used for description. It should be noted that FIG. 1 merely shows some components included in the electronic device 1000 by using an example, and actual sizes, actual positions, and actual structures of these components are not limited by the figure. The following accompanying drawings also merely show some components by using examples, and actual sizes, actual positions, and actual structures of these components are not limited by the following accompanying drawings. Details are not described below again.

As shown in FIG. 1, the electronic device 1000 may include a watch body 1001 and watch straps 1002. The watch straps 1002 are connected to the watch body 1001. For example, there may be two watch straps 1002. The two watch straps 1002 are respectively connected to two ends of the watch body 1001. When a user wears the electronic device 1000, the watch straps 1002 may be configured to fasten the watch body 1001 to the user. In another implementation, there may alternatively be one watch strap 1002.

The watch body 1001 may include a health monitoring module 100, a screen 210, and a housing 200. The health monitoring module 100 may be mounted on the housing 200. The health monitoring module 100 may be configured to monitor a health parameter indicator of the user. In this way, users can monitor their health statuses at any time. For example, the health monitoring module 100 may be configured to measure blood pressure and electrocardiogram data of the user.

In some implementations, the screen 210 may be configured to display an image and the like. The screen 210 may be a flat screen or a curved screen. A display of the screen 210 may be an organic light-emitting diode (organic light-emitting diode, OLED) display, an active-matrix organic light-emitting diode (active-matrix organic light-emitting diode, AMOLED) display, a liquid crystal display (liquid crystal display, LCD), or the like.

For example, the housing 200 may include a middle frame 220 and a rear cover 230. The screen 210 and the rear cover 230 are spaced apart and disposed oppositely, and the middle frame 220 is connected between the screen 210 and the rear cover 230. The screen 210, the middle frame 220, and the rear cover 230 jointly enclose internal space 1003 of the electronic device 1000. The internal space 1003 of the electronic device 1000 may be configured to place a component of the electronic device 1000, for example, a mainboard, a battery, a speaker, or a microphone. In another implementation, the middle frame 220 and the rear cover 230 may alternatively be an integrated mechanical part. When the user wears the electronic device 1000, the screen 210 is located on a side that is of the watch body 1001 and that faces away from the wrist skin of the user, the rear cover 230 is located on a side that is of the watch body 1001 and that faces the wrist skin of the user, and the rear cover 230 may be in contact with the wrist skin of the user.

FIG. 2 is a partial sectional view of the electronic device 1000 shown in FIG. 1 along a section line A-A in an implementation.

As shown in FIG. 1 and FIG. 2, the health monitoring module 100 may be disposed on a side face of the electronic device 1000. The side face of the electronic device 1000 may surround a front face of the electronic device 1000 (a face facing the user when the electronic device 1000 is normally used). For an electronic device 1000 having a display, such as a smartwatch or a smart band, a front face is a face on which the screen 210 is located, and a side face of the electronic device 1000 may surround the screen 210. For an electronic device 1000 without the screen 210, a front face may be a main user interface of the electronic device 1000, and a side face of the electronic device 1000 may surround the main user interface.

For example, when the housing 200 includes the middle frame 220 and the rear cover 230, the health monitoring module 100 may be mounted on the middle frame 220, and protrudes from an outer surface 2201 of the middle frame 220. The outer surface 2201 of the middle frame 220 is a surface that is of the middle frame 220 and that is away from the internal space 1003 of the electronic device 1000, that is, the side face of the electronic device 1000. It may be understood that the health monitoring module 100 is mounted on the side face of the electronic device 1000, to facilitate touching or pressing by the user. In comparison with a solution in which the health monitoring module 100 is mounted on the screen 210, mounting the health monitoring module 100 on the side face of the electronic device 1000 does not need to occupy a display region area of the screen 210. This helps increase a display area of the electronic device 1000.

In addition, the health monitoring module 100 may alternatively be electrically connected to a circuit located inside the electronic device 1000. For example, when the electronic device 1000 includes a main board and a sub-board, the health monitoring module 100 may be electrically connected to the main board and/or the sub-board, to transmit a signal. When the electronic device 1000 includes only the mainboard, the health monitoring module 100 may be electrically connected to the mainboard.

In some implementations, the electronic device 1000 may further include a processor (not shown in the figure). The processor may be fastened to the mainboard, and is electrically connected to the mainboard. The health monitoring module 100 may be electrically connected to the processor. A signal collected by the health monitoring module 100 may be transmitted to the processor via a flexible printed circuit board 99 and the mainboard.

In some implementations, the processor may be configured to determine, based on comprehensive analysis of a plurality of health parameters of the user detected by the health monitoring module 100, whether a current physical status of the user is healthy. If an analysis result is that the user is currently in a suboptimal health status, the processor may further provide adjustment suggestion for a routine lifestyle, dietary habits, and the like of the user based on the analysis result. In some implementations, the processor may be further configured to: analyze a current psychological state of the user based on the parameters detected by the health monitoring module 100, and determine whether the user has a mental health problem such as depression.

In some implementations, when the electronic device 1000 is the watch or the band, the health monitoring module 100 may alternatively be a button of the watch or the band. In this way, the user may further perform an operation on the watch or the band by touching or pressing the health monitoring module 10, to interact with the electronic device 1000. It may be understood that, a health monitoring function and a button function of the electronic device are integrated into the health monitoring module 100, so that space in which the button is additionally disposed can be saved for the electronic device 1000, thereby facilitating miniaturization of the electronic device 1000.

In some implementations, the health monitoring module 100 may be a touch-sensitive button. When the button function is implemented, the health monitoring module 100 does not need to move relative to the middle frame 220, and the user only needs to touch the health monitoring module 100 to implement the button function.

In another implementation, the health monitoring module 100 may alternatively be a key with a stroke. When the button function is implemented, the user needs to press the health monitoring module 100, so that the health monitoring module 100 moves relative to the middle frame 220, and specific displacement is generated. In this way, the button function can be implemented.

For ease of description, a thickness direction of the health monitoring module 100 is defined as a Z-axis; a length direction of the health monitoring module 100 is defined as a Y-axis; and a width direction of the health monitoring module 100 is defined as an X-axis. It may be understood that a coordinate system may be flexibly set based on a specific actual requirement.

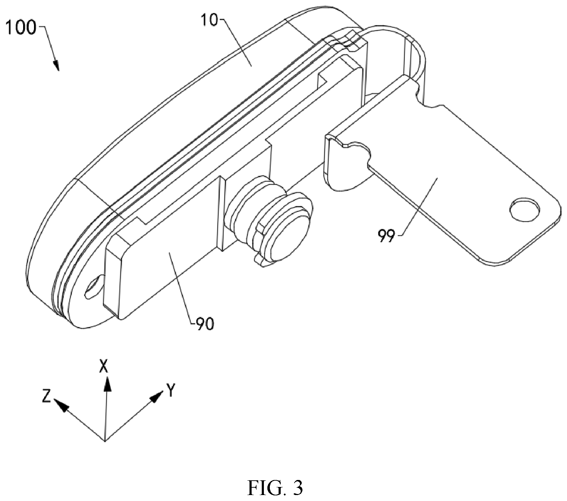

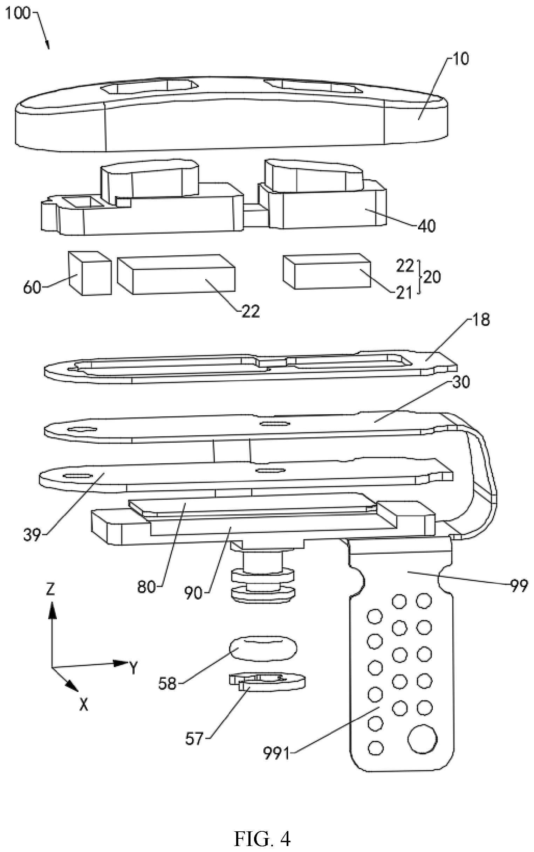

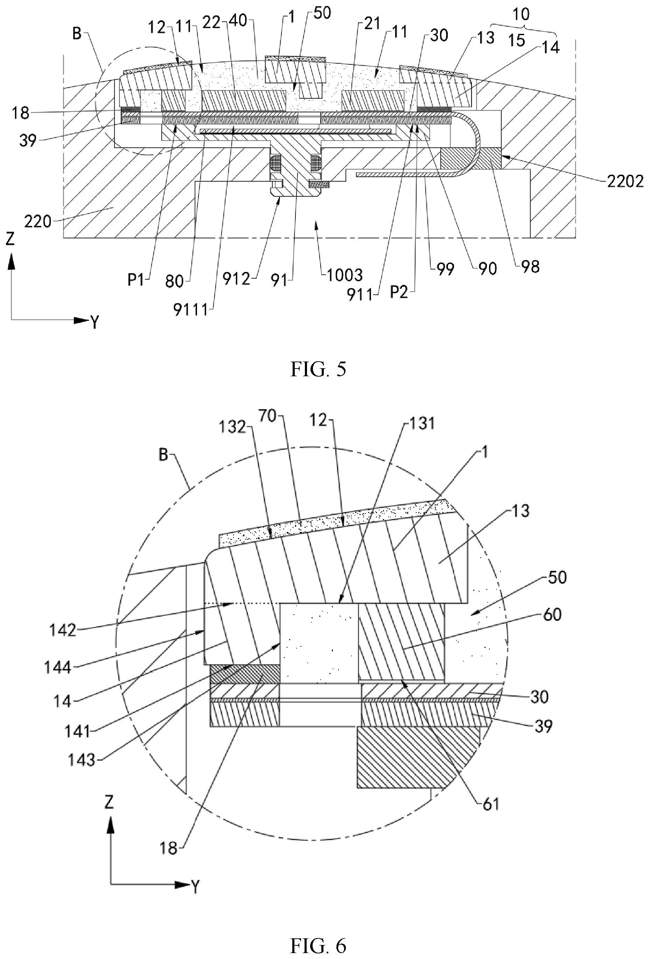

FIG. 3 is a diagram of a structure of the health monitoring module 100 shown in FIG. 1 in an implementation. FIG. 4 is an exploded diagram of the health monitoring module 100 shown in FIG. 3 in an implementation. FIG. 5 is a partial sectional view of the electronic device 1000 shown in FIG. 1 along a section line A-A in another implementation.

As shown in FIG. 3, FIG. 4, and FIG. 5, the health monitoring module 100 may include an enclosure 10, an optical heart rate module 20, a circuit board 30, and a light-transmitting member 40. The enclosure 10 is fastened to a side of the circuit board 30, and the enclosure 10 and the circuit board 30 jointly enclose accommodation space 50. Light-transmitting holes 11 are disposed in the enclosure 10, and the light-transmitting holes 11 communicate the accommodation space 50 with the outside. The optical heart rate module 20 is located in the accommodation space 50, and the optical heart rate module 20 is fastened to the circuit board 30. The light-transmitting member 40 is fastened to the enclosure 10 and at least partially filled in the light-transmitting holes 11. The following describes in detail the enclosure 10, the optical heart rate module 20, the circuit board 30, and the light-transmitting member 40 with reference to the accompanying drawings.

For example, the enclosure 10 may include a conductive portion 1, the conductive portion 1 is made of a conductive material, a part of the conductive portion 1 protrudes from a top face 12 of the enclosure 10, and another part is electrically connected to the circuit board 30. The top face 12 of the enclosure 10 is a surface of a side that is of the enclosure 10 and that is away from the circuit board 30. When the health monitoring module 100 is mounted on the middle frame 220, the top face 12 of the enclosure 10 may protrude from the middle frame 220 (as shown in FIG. 5), and the part of the conductive portion 1 protrudes from the top face 12 of the enclosure 10. A finger may be in contact with the top face 12 of the enclosure 10, and is in an electrical connection to the conductive portion 1, so that the enclosure 10 can collect an electrical signal of a user.

For example, when the user wears the electronic device 1000, the top face 12 of the enclosure 10 of the health monitoring module 100 is an externally exposed structure. When the user needs to measure an electrocardiogram, the finger may be in contact with the top face 12 of the enclosure 10, and is in the electrical connection to the conductive portion 1, so that the enclosure 10 can collect the electrical signal of the user. An electrocardiogram (Electrocardiogram, ECG) waveform of the user can be obtained through processing of the electrical signal. The electrocardiogram waveform can reflect a cardiac function status of the user.

In some implementations, the enclosure 10 may be further configured to sense or detect touch or pressing action of the user, so that the health monitoring module 100 has a button function. For example, when the user touches or presses the conductive portion 1, the conductive portion 1 may collect the electrical signal of the user. It is determined, based on whether the conductive portion 1 collects the electrical signal of the user, whether the user touches or presses the conductive portion.

In another implementation, the health monitoring module 100 may alternatively be configured to sense, via another module, the touch or pressing action performed by the user on the enclosure. For example, the optical heart rate module 20 may alternatively be configured to sense the touch action performed by the user on the enclosure.

FIG. 6 is an enlarged diagram of a structure shown in FIG. 5 at B in an implementation.

As shown in FIG. 5 and FIG. 6, the enclosure 10 may include a top plate 13 and a side wall 14 (for ease of understanding, the top plate 13 and the side wall 14 are schematically distinguished based on dashed lines in FIG. 5 and FIG. 6). The top plate 13 includes a first face 131 and a second face 132 that are disposed back to back. The side wall 14 includes a first end face 141, a second end face 142, an inner side face 143, and an outer side face 144. The first end face 141 and the second end face 142 are disposed back to back. The inner side face 143 is connected between the first end face 141 and the second end face 142. The outer side face 144 is connected between the first end face 141 and the second end face 142. The second end face 142 of the side wall 14 is fastened to the first face 131 of the top plate 13. It may be understood that the second face 132 of the top plate 13 forms the top face 12 of the enclosure 10. The first end face 141 of the side wall 14 is a bottom face of the enclosure 10.

In other words, as shown in FIG. 6, the first face 131 and the second face 132 are sequentially disposed from bottom to top in a Z-axis direction. The outer side face 144 and the inner side face 143 are sequentially disposed from left to right in a Y-axis direction. The first end face 141 and the second end face 142 are sequentially disposed from bottom to top in the Z-axis direction.

The light-transmitting holes 11 may be disposed in the enclosure 10. For example, the light-transmitting holes 11 may be located on the top plate 13. The light-transmitting holes 11 penetrate the first face 131 and the second face 132 of the top plate 13.

In some implementations, the top plate 13 and the side wall 14 may be an integrally formed mechanical part. It should be noted that, obtaining an integrated mechanical part of two parts by using an integral forming process means that, in a process of forming either of the two parts, the part is connected to the other part, and the two parts are connected without secondary processing (for example, adhesive bonding, welding, snap-fit connections, or screw connections). For example, the top plate 13 and the side wall 14 may be integrally formed by using a potting process. In some implementations, the top plate 13 and the side wall 14 may alternatively be separately manufactured, and are fastened in a manner of adhesive bonding or the like.

There may be a plurality of implementations for the conductive portion 1 of the enclosure 10. The enclosure 10 may be entirely used as the conductive portion 1, or may be partially used as the conductive portion 1.

In some implementations, the enclosure 10 may be entirely used as the conductive portion 1. For example, the enclosure 10 may be entirely made of a metal material. The metal material may be conductive, and the entire enclosure 10 may be conductive. When the user is in contact with the enclosure 10, any position on the top face 12 of the enclosure 10 may be electrically connected to the user. An area of the electrical connection between the enclosure 10 and the user is large, and reliability of the electrical connection is good. In addition, in comparison with a material such as plastic, the metal material has better strength and is wear-resistant, and the enclosure 10 has better overall strength and a longer service life. When the enclosure 10 is entirely made of the metal material, the enclosure 10 may be processed by a computer numerical control (Computer numerical control, CNC) machine or directly molded. Alternatively, the enclosure 10 may be divided into a plurality of parts for separate preparation, and finally, the plurality of parts are assembled to form the enclosure 10. This is not limited in this application. In another implementation, the enclosure 10 may alternatively be made of another non-metal conductive material.

For example, when the enclosure 10 is entirely made of the metal material, the enclosure 10 may be directly fastened to the circuit board 30 through a conductive adhesive, and is electrically connected to the circuit board 30. Alternatively, the enclosure 10 may be directly fastened to the circuit board 30 through welding, and is electrically connected to the circuit board 30. It may be understood that the conductive adhesive has a conductive function, and further has a bonding function. The enclosure 10 is fastened to the circuit board 30 through the conductive adhesive, and the conductive adhesive may be used for electrical conduction between the enclosure 10 and the circuit board 30, so that an electrical connection structure between the enclosure 10 and the circuit board 30 may be omitted, thereby helping reduce a size of the health monitoring module 100.

In some other implementations, a part of the top plate 13 of the enclosure 10 and a part of the side wall 14 of the enclosure 10 form the conductive portion 1. In this case, the conductive portion 1 is made of the conductive material, and a part of the enclosure 10 other than the conductive portion 1 may be made of an insulation material. For example, the conductive portion 1 is made of the metal material, and the another part is made of a ceramic material. The part made of the metal material and the part made of the ceramic material may be fastened in the manner of adhesive bonding or the like. Alternatively, the conductive portion 1 is made of the metal material, and the another part is made of a polymeric inorganic material. In a process of preparing the enclosure 10, the conductive portion 1 may be first prepared, the conductive portion 1 is used as an insert, and the enclosure 10 is formed by using an insert injection molding process.

In some other implementations, the top plate 13 of the enclosure 10 may form the conductive portion 1. The side wall 14 of the enclosure 10 may be made of an insulation material.

As shown in FIG. 6, the health monitoring module 100 further includes a conducting layer 70 disposed on the top face 12 of the enclosure 10. The conducting layer 70 may be electrically connected to the conductive portion 1, and material hardness of the conducting layer 70 may be greater than material hardness of the conductive portion 1. When the user needs to measure the electrocardiogram, the finger may be in contact with the conducting layer 70. The electrical signal of the user may be transmitted to the conductive portion 1 through the conducting layer 70. It may be understood that, the conducting layer 70 may be configured to prevent the collection of the electrical signal of the user from being affected by adverse factors such as a scratch, corrosion, and collision on the top face 12 of the enclosure 10.

In some implementations, the conducting layer 70 may be formed by using a physical vapor deposition (physical vapor deposition, PVD) process. In this way, the conducting layer 70 may feature high hardness, high wear resistance, and high corrosion resistance. In another implementation, a technology such as diamond-like carbon (diamond-like carbon) may be further selected for the conducting layer 70, to obtain higher hardness.

In some implementations, the conducting layer 70 may be made of a material such as chromium or a chromium alloy.

In another implementation, when the material of the conducting layer 70 is a transparent conducting material such as indium tin oxide (Indium Tin Oxide, ITO), the conducting layer 70 may be further fastened to a surface of a side that is of the light-transmitting member 40 and that is away from the circuit board 30.

As shown in FIG. 5, the top face 12 of the enclosure 10 may include an arc surface. In this way, the user has a good tactile feeling when touching the enclosure 10.

In some implementations, a height of the enclosure 10 in the Z-axis direction may be within a range of 1 millimeter (millimeter, mm) to 3 mm. For example, the height of the enclosure 10 in the Z-axis direction may be 1 mm, 1.5 mm, 2 mm, 2.8 mm, or 3 mm. In this way, strength of the enclosure 10 is good.

In some implementations, a length of the enclosure 10 in the Y-axis direction may be within a range of 6 mm to 20 mm. For example, the length of the enclosure 10 in the Y-axis direction may be 6 mm, 8 mm, 14 mm, 18 mm, or 20 mm.

In some implementations, the circuit board 30 may be a rigid printed circuit board (Printed Circuit Board, PCB), or may be a flexible printed circuit (Flexible Printed Circuit, FPC) board.

In some implementations, the health monitoring module 100 may further include a reinforcement plate 39. The reinforcement plate 39 may be connected to a side that is of the circuit board 30 and that is away from the optical heart rate module 20. The reinforcement plate 39 may be configured to reinforce the circuit board 30. For example, the reinforcement plate 39 may be made of a metal material, for example, iron or an iron alloy.

As shown in FIG. 5, the optical heart rate module 20 may be fastened to the circuit board 30, and is electrically connected to the circuit board 30. For example, the optical heart rate module 20 may include a light emitter 21 and a light receiver 22. The light emitter 21 and the light receiver 22 are spaced apart on the circuit board 30. The light emitter 21 may be configured to emit light beams. The light receiver 22 is configured to: receive the light beams, and convert an optical signal into an electrical signal. It may be understood that, the light emitter 21 and the light receiver 22 shown in FIG. 5 are merely exemplary representations. In another implementation, positions of the light emitter 21 and the light receiver 22 may alternatively be exchanged.

In some implementations, the circuit board 30 may be configured to electrically connect to a mainboard (not shown in the figure). After the conductive portion 1 of the enclosure 10 collects the electrical signal of the user, the electrical signal may be transmitted to the mainboard via the circuit board 30. For example, the health monitoring module 100 may further include the flexible printed circuit board 99. The flexible printed circuit board 99 may be configured to transmit a signal on the circuit board 30 to the mainboard. Details are described below.

As shown in FIG. 5 and FIG. 6, the enclosure 10 may be fastened to the side of the circuit board 30. The enclosure 10 and the circuit board 30 jointly enclose the accommodation space 50. The light-transmitting holes 11 communicate the accommodation space 50 with the outside. For example, the first end face 141 of the side wall 14 is fastened to the circuit board 30. The inner side face 143 of the side wall 14, the first face 131 of the top plate 13, and the circuit board 30 may jointly enclose the accommodation space 50. The light-transmitting holes 11 may penetrate the first face 131 and the second face 132 of the top plate 13. In this way, the light-transmitting holes 11 can communicate the accommodation space 50 with the outside.

In some implementations, the first end face 141 of the side wall 14 may be fastened to the circuit board 30 through a waterproof adhesive 18.

As shown in FIG. 4 and FIG. 5, the waterproof adhesive may be of an annular shape. When the enclosure 10 is fastened to the circuit board 30 through the waterproof adhesive 18, the waterproof adhesive 18 can seal the accommodation space 50. In addition, when the health monitoring module 100 is located in a humid environment or underwater, the waterproof adhesive 18 can further prevent water vapor from entering the accommodation space 50 from a gap between the enclosure 10 and the circuit board 30, to implement a waterproof function for a component (for example, the optical heart rate module 20) in the accommodation space 50.

In another implementation, the first end face 141 of the side wall 14 may be further fastened to the circuit board 30 through welding.

As shown in FIG. 5, the optical heart rate module 20 is located in the accommodation space 50. The optical heart rate module 20 and the light-transmitting holes 11 are disposed oppositely. The optical heart rate module 20 may emit and receive light beams through the light-transmitting holes 11. The optical heart rate module 20 may be configured to collect a photoplethysmogram (photoplethysmogram, PPG) signal of the user. For example, the light emitter 21 and the light receiver 22 may be located in the accommodation space 50. The light emitter 21 and the light-transmitting hole 11 may be disposed oppositely. The light receiver 22 and the light-transmitting hole 11 may be disposed oppositely. When the finger of the user presses the enclosure 10, the finger of the user may cover the light-transmitting holes 11, and the light beams emitted by the light emitter 21 may irradiate the finger of the user through the light-transmitting hole 11. The light beams may be reflected or scattered by blood vessels of the finger of the user, and return to the accommodation space 50 through the light-transmitting hole 11. The light beams are received by the light receiver 22. An electrical signal is formed through optical-to-electrical conversion, and is transmitted to the circuit board 30.

For example, the light beams reflected or scattered by the blood vessels of the finger of the user may reflect blood flow information of the user. Blood flow usually refers to flow of blood in blood vessels of a body. The blood flow is a basic parameter of a blood circulatory system and reflects flow of the blood through arteries, capillaries, and veins under action of pumping of the blood by the heart. The blood flow may be represented based on a plurality of parameters, including but not limited to a speed, a flow rate, a direction, stability (whether the blood flows stably in the circulatory system), resistance (the blood flow is affected by resistance of blood vessel walls), pulsatility (fluctuation of the blood flow in a cardiac pulsation process), distribution (distribution of the blood flow in the body), and the like. The blood flow information is measured and analyzed to evaluate a function of a cardiovascular system, diagnose cardiovascular diseases, and develop effective treatment plans.

The blood flow information and other physiological features such as a heart rate, blood oxygen, and breathing are associated with each other and affect each other. In physiological feature monitoring, comprehensive consideration of the information helps understand physiological and health statuses of the body more accurately.

In some implementations, there may be two light-transmitting holes 11, the two light-transmitting holes 11 are spaced apart, and the light emitter 21 and the light receiver 22 are respectively disposed in correspondence with the two light-transmitting holes 11. For example, for ease of description, the two light-transmitting holes 11 are: a first light-transmitting hole 111 and a second light-transmitting hole 112. The first light-transmitting hole 111 and the second light-transmitting hole 112 are spaced apart. The first light-transmitting hole 111 and the light emitter 21 are disposed oppositely, and the second light-transmitting hole 112 and the light receiver 22 are disposed oppositely. A part of the light-transmitting member 40 is filled in the first light-transmitting hole 111, and a part of the light-transmitting member 40 is filled in the second light-transmitting hole 112. The light emitter 21 emits the light beams through the first light-transmitting hole 111 and the light-transmitting member 40. The light receiver 22 receives the light beams through the second light-transmitting hole 112 and the light-transmitting member 40. In another implementation, there may alternatively be one light-transmitting hole 11.

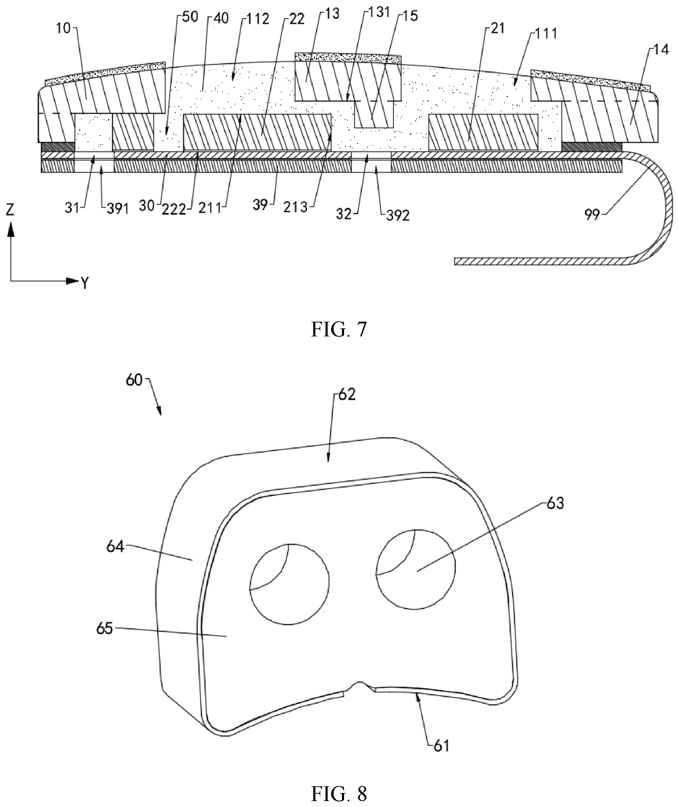

FIG. 7 is a diagram of a partial structure of a structure shown in FIG. 5 in an implementation.

As shown in FIG. 5 and FIG. 7, the optical heart rate module 20 and the enclosure 10 may be spaced. For example, the light emitter 21 may include a top face 211, a bottom face 212, and a side face 213. The top face 211 of the light emitter 21 and the bottom face 212 of the light emitter 21 are disposed back to back. The side face 213 of the light emitter 21 is connected between the top face 211 of the light emitter 21 and the bottom face 212 of the light emitter 21. The bottom face 212 of the light emitter 21 is connected to the circuit board 30. The top face 211 of the light emitter 21 faces the light-transmitting holes 11. For a position relationship between the light receiver 22 and the enclosure 10, refer to the light emitter 21. Details are not described herein again.

It may be understood that the optical heart rate module 20 and the enclosure 10 are spaced apart, and a gap between the enclosure 10 and the optical heart rate module 20 may be used to dispose a waterproof structure, to perform waterproof and dustproof protection on the optical heart rate module 20. In addition, the gap between the enclosure 10 and the optical heart rate module 20 may be further used to accommodate an assembly error, to avoid damage to either of the enclosure 10 or the optical heart rate module 20 caused by abutting or collision of the enclosure 10 and the optical heart rate module 20 in an assembly process.

In another implementation, the optical heart rate module 20 may alternatively be connected to the enclosure 10. For example, the side face 213 of the light emitter 21 may be connected to the enclosure 10.

In some implementations, the light emitter 21 and a part of the enclosure 10 may be disposed oppositely in the Z-axis direction. To be specific, in the thickness direction of the health monitoring module 100, projection of the light emitter 21 on the circuit board 30 may partially overlap projection of the enclosure 10 on the circuit board 30. The light emitter 21 does not need to be disposed to be directly opposite to the light-transmitting hole 11. It may be understood that, a light source of the light emitter 21 may be located on a side of the light emitter 21, instead of being located at a center of the light emitter 21. The center of the light emitter 21 and a center of the light-transmitting hole 11 are staggered, so that the light source of the light emitter 21 and the center of the light-transmitting hole 11 may be disposed oppositely, and light beams emitted by the light source irradiate the skin or tissue of the user through the light-transmitting hole 11 as much as possible. In another implementation, the light source of the light emitter 21 may alternatively be located at a central position of the light emitter 21. A person skilled in the art may design a relative position relationship between the light emitter 21, the light-transmitting hole 11, and the enclosure 10 based on a specific position of the light source of the light emitter 21 on the light emitter 21, so that the light beams emitted by the light source irradiate the skin of the user through the light-transmitting hole 11 as much as possible.

For example, the light emitter 21 may include one light source, or may include a plurality of light sources. When the light emitter 21 includes the plurality of light sources, wavelengths of light beams emitted by these light sources may be different from each other, or wavelengths of these light sources are different from each other. For example, the wavelengths of the plurality of light sources may include a near-infrared light band, a red light band, a blue light band, a yellow light band, a green light band, and the like.

In some implementations, the height of the enclosure 10 in the Z-axis direction may be within the range of 1 mm to 3 mm. In a process in which the light emitter 21 emits the light beams, the height of the enclosure 10 is high, to provide a specific distance for the light emitter 21 to emit the light beams, so that the light beams can irradiate a large area of the skin surface of the user.

In some implementations, the enclosure 10 may further include a light-shielding portion 15 (for ease of understanding, the top plate 13, the side wall 14, and the light-shielding portion 15 are schematically distinguished based on dashed lines in FIG. 5). The light-shielding portion 15 may be connected to the first face 131 of the top plate 13, and is located in the accommodation space 50. The light-shielding portion 15 is at least partially located between the light emitter 21 and the light receiver 22. The light-shielding portion 15 is made of an opaque material. In this way, the light-shielding portion 15 may block the light beams emitted by the light emitter 21, and reduce a probability that the light beams emitted by the light emitter 21 are directly propagated to a position of the light receiver 22 in the accommodation space 50, so that the light receiver 22 receives, as much as possible, light beams that are all reflected by the user, thereby improving measurement accuracy of the PPG signal. The top plate 13 and the side wall 14 may form a frame portion 19 of the enclosure 10. The frame portion 19 may be connected to the circuit board 30, and the frame portion 19 and the circuit board 30 enclose the accommodation space 50.

In some implementations, the light-shielding portion 15 and the top plate 13 may be an integrally formed mechanical part. For example, the light-shielding portion 15, the top plate 13, and the side wall 14 may be integrally formed through CNC or mold processing.

In some implementations, the light-shielding portion 15 and the circuit board 30 may be spaced. For example, in the Z-axis direction, a spacing between the light-shielding portion 15 and the circuit board 30 may be within a range of 0.2 mm to 0.6 mm. For example, in the Z-axis direction, a spacing between the light-shielding portion 15 and the circuit board 30 may be 0.2 mm, 0.3 mm, or 0.6 mm. In this way, a gap between the light-shielding portion 15 and the circuit board 30 is small, and light-shielding effect of the light-shielding portion 15 is good.

In another implementation, the light-shielding portion 15 may further be connected to the circuit board 30. The light-shielding portion 15 divides the accommodation space 50 into two pieces of independent spaces.

As shown in FIG. 5 and FIG. 7, the light-transmitting member 40 may be fastened to the enclosure 10 and at least partially filled in the light-transmitting holes 11. It may be understood that the light-transmitting member 40 is configured to seal the light-transmitting holes 11, to prevent external impurities such as the dust from interfering with operation of the optical heart rate module 20 by entering the accommodation space 50 through the light-transmitting holes 11.

For example, the light-transmitting member 40 may be formed through curing of a waterproof adhesive. In this way, the enclosure 10, the circuit board 30, and the light-transmitting member 40 can cooperate with each other to perform waterproof protection for the optical heart rate module 20, to prevent external impurities such as the water vapor from interfering with operation of the optical heart rate module 20 by entering the accommodation space 50 through the light-transmitting holes 11. The waterproof structure of the optical heart rate module 20 does not need to be additionally disposed. This facilitates miniaturization of the health monitoring module 100. An overall waterproof function of the health monitoring module 100 is good. In some implementations, when the health monitoring module 100 is used as an independent product, a waterproof level of 5 ATM may be reached, to meet a requirement of the user for using the electronic device 1000 in an environment with high humidity.

In some implementations, a top face of the light-transmitting member 40 is an arc surface. The top face of the light-transmitting member 40 is the surface of the side that is of the light-transmitting member 40 and that is away from the circuit board 30. When the light-transmitting member 40 protrudes from the enclosure 10, a height difference between the top face of the light-transmitting member 40 and the top face 12 of the enclosure may be set to be small, and transition can be smooth between the top face of the light-transmitting member 40 and the top face 12 of the enclosure. The user has the good tactile feeling when touching the enclosure 10.

The light-transmitting member 40 is made of a transparent material. For example, the light-transmitting member 40 may be transparent glass, or may be formed after transparent glue is cured. In this way, the light-transmitting member 40 neither affects that the light beams emitted by the light emitter 21 irradiate the skin or tissue of the user through the light-transmitting hole 11, nor affects that the reflected light beams are received by the light receiver 22 through the light-transmitting hole 11.

In some implementations, the light-transmitting member 40 may be filled in both the light-transmitting holes 11 and the accommodation space 50. The light-transmitting member 40 is in contact with and fastened to the enclosure 10, the optical heart rate module 20 (including the light receiver 22 and the light emitter 21), and the circuit board 30. For example, the optical heart rate module 20 (including the light receiver 22 and the light emitter 21) and the enclosure 10 may be spaced, and the light-transmitting member 40 may be in contact with and connected to the top face 211 and the side face 213 of the light emitter 21. The light-transmitting member 40 may also be in contact with and connected to a top face and a side face of the light receiver 22. The light-transmitting member 40 may be in contact with and fastened to a surface of a side that is of the circuit board 30 and that is close to the enclosure 10. In this way, the light-transmitting member 40 can wrap the optical heart rate module 20 (including the light receiver 22 and the light emitter 21). The light-transmitting member 40 can implement a waterproofing function for the optical heart rate module 20.

In another implementation, when the side face 213 of the light emitter 21 is connected to the enclosure 10, the light-transmitting member 40 may be connected to the top face 211 of the light emitter 21.

In another implementation, the light-transmitting member 40 may further be filled in a part of the accommodation space 50. The light-transmitting member 40 may be in contact with and fastened to the enclosure 10. In addition, the light-transmitting member 40, the optical heart rate module 20 (including the light receiver 22 and the light emitter 21), and the circuit board 30 are spaced apart.

In another implementation, the light-transmitting member 40 may alternatively be located only in the light-transmitting holes 11. In this way, the light-transmitting member 40 can implement waterproof, dustproof, and light transmission functions. In this case, the light-transmitting member 40 is in contact with and fastened to the enclosure 10. The light-transmitting member 40, the circuit board 30, and the optical heart rate module 20 (the light receiver 22 and the light emitter 21) are spaced apart.

In some implementations, the enclosure 10 and the light-transmitting member 40 may be an integrally formed mechanical part. In this way, connection strength between the enclosure 10 and the light-transmitting member 40 is good. For example, when the light-transmitting member 40 is formed through curing of glue, the transparent glue may be injected into the enclosure 10 via a jig. After the glue is cured, the light-transmitting member 40 is directly fastened to the enclosure 10 to form the mechanical part. In this way, a connection structure does not need to be additionally disposed between the light-transmitting member 40 and the enclosure 10 for the health monitoring module 100. This simplifies a technical process and helps reduce costs of the health monitoring module 100. In addition, the light-transmitting member 40 is formed by using a potting process via the jig, no additional connection structure needs to be disposed between the enclosure 10 and the light-transmitting member 40, and there is no visual segment gap between the enclosure 10 and the light-transmitting member 40, providing good appearance.

In some implementations, a first through hole 31 and a second through hole 32 that are spaced apart may be disposed on the circuit board 30. Both the first through hole 31 and the second through hole 32 may communicate the accommodation space 50 with the outside. The light emitter 21, the first through hole 31, and the second through hole 32 may be spaced. The light receiver 22, the first through hole 31, and the second through hole 32 may be spaced. When the light-transmitting member 40 may be formed by using the potting process, either the first through hole 31 or the second through hole 32 may be used as a glue filling hole, and the other may be used as a glue outlet hole.

In some implementations, the light-transmitting member 40 may be partially filled in the light-transmitting holes 11, partially filled in the accommodation space 50, partially filled in the first through hole 31, and partially filled in the second through hole 32.

For example, assembling steps of the health monitoring module 100 may include: fastening the optical heart rate module 20 to the circuit board 30, and fastening the enclosure 10 to the circuit board 30 in advance through the waterproof adhesive 18, to form the accommodation space 50. The light-transmitting holes 11 are sealed on a side of the top face 12 of the enclosure 10 via the jig. The first through hole 31 and the second through hole 32 that are spaced apart may be disposed on the circuit board 30, and both the first through hole 31 and the second through hole 32 communicate the accommodation space 50 with the outside. The glue is injected into the accommodation space 50 through the first through hole 31/the second through hole, and the glue is cured to form the light-transmitting member 40. The light-transmitting member 40 may simultaneously be in contact with and connected to the enclosure 10, the optical heart rate module 20 (including the light receiver 22 and the light emitter 21), and the circuit board 30, to form the mechanical part integrally formed by the enclosure 10, the light-transmitting member 40, the optical heart rate module 20, and the circuit board 30. In this case, the light-transmitting member 40 may be further configured to enhance connection strength between the circuit board 30 and the enclosure 10.

It may be understood that, the light-transmitting member 40 forms the mechanical part integrally formed by the enclosure 10, the optical heart rate module 20, and the circuit board 30 by using the waterproof adhesive injection process, and the light-transmitting member 40 can form good waterproof protection for the optical heart rate module 20.

In some implementations, when the light-shielding portion 15 of the enclosure 10 divides the accommodation space 50 into the two pieces of independent space, the light-transmitting member 40 may also include two spaced parts. When the light-transmitting member 40 is assembled with the enclosure 10 and the circuit board 30 by using the potting process via the jig, potting may be separately performed on two independent cavities included in the accommodation space 50.

In some implementations, when the health monitoring module 100 is mounted on the side face of the electronic device 1000, a width of the health monitoring module 100 (a length of the health monitoring module 100 in the X-axis direction) needs to be set to be small, to be applicable to a thin electronic device. For example, the first through hole 31, the second through hole 32, the light emitter 21, and the light receiver 22 may be spaced in the length direction of the health monitoring module 100. In this way, in comparison with a solution in which the first through hole 31 and the light emitter 21 are spaced apart in the width direction of the health monitoring module, that the first through hole 31, the second through hole 32, the light emitter 21, and the light receiver 22 are spaced apart in the length direction of the health monitoring module 100 helps reduce the width of the health monitoring module 100.

For example, as shown in FIG. 1 and FIG. 7, the flexible printed circuit board 99 may be connected to a left side or a right side of the circuit board 30. For example, the flexible printed circuit board 99 may be connected to the right side of the circuit board 30. In comparison with that the flexible printed circuit board 99 is connected to a side that is of the circuit board 30 and that is close to the screen 210, or the flexible printed circuit board 99 is connected to a side that is of the circuit board 30 and that is close to the rear cover 230, the solution in this embodiment helps reduce the width of the health monitoring module 100.

As shown in FIG. 7, the light emitter 21 may be located between the first through hole 31 and the second through hole 32, or the light receiver 22 may be located between the first through hole 31 and the second through hole 32. For example, the first through hole 31 may be located on the left side of the light receiver 22, and the second through hole 32 is located between the light emitter 21 and the light receiver 22.

It may be understood that, in comparison with a solution in which both the light emitter 21 and the light receiver 22 are located between the first through hole 31 and the second through hole 32, the light emitter 21 is located between the first through hole 31 and the second through hole 32, or the light receiver 22 is located between the first through hole 31 and the second through hole 32. Large tracing space may be reserved on a right side of the light emitter 21 or on the left side of the light receiver 22. The tracing space may be used to arrange a signal transmission line, and transmit signals of the conductive portion 1 and the light receiver 22 to the flexible printed circuit board 99. Further, the flexible printed circuit board 99 may be connected to the left side or the right side of the circuit board 30.

In some implementations, when the first through hole 31 and the second through hole 32 are disposed on the circuit board 30, a third through hole 391 and a fourth through hole 392 may alternatively be disposed on the reinforcement plate 39 correspondingly. The third through hole 391 and the first through hole 31 are disposed oppositely and communicate with each other. The fourth through hole 392 and the second through hole 32 are disposed oppositely and communicate with each other. The light-transmitting member 40 may be filled in the light-transmitting holes 11, the accommodation space 50, the first through hole 31, the second through hole 32, the third through hole 391, and the fourth through hole 392.

In another implementation, the light-transmitting member 40 may alternatively be formed by using an injection molding process in low temperature and low pressure conditions. Either the first through hole 31 or the second through hole 32 may be used as a plastic injection hole, and the other may be used as a plastic outlet hole.

In another implementation, the light-transmitting holes 11 are sealed on the side of the top face 12 of the enclosure 10 via the jig, and the transparent glue is injected into the light-transmitting holes 11 of the enclosure 10. After the glue is cured, the mechanical part integrally formed by the enclosure 10 and the light-transmitting member 40 is formed. The optical heart rate module 20 is fastened to the circuit board 30, and the enclosure 10 is fastened to the circuit board 30, to complete assembly of the enclosure 10, the optical heart rate module 20, the circuit board 30, and the light-transmitting member 40.

In another implementation, the light-transmitting member 40 may alternatively be first manufactured and formed through processing, and fastened to the enclosure 10 by using a process such as adhesive bonding, snap-fit connections, or welding.

In some implementations, hardness of the light-transmitting member 40 may meet a pencil hardness test of more than 2H. In this way, the hardness of the light-transmitting member 40 is good, and a surface that is of the light-transmitting member 40 and that is away from the circuit board 30 is not easily scratched, which has good aesthetics. In addition, the light-transmitting member 40 has little impact on the light beams. When the light emitter 21 emits the light beams, and the light receiver 22 receives the light beams via the light-transmitting member 40, there are a few light beam losses, and measurement accuracy of the optical heart rate module 20 is high.

In some implementations, a transmittance of the light-transmitting member 40 may be greater than 90%. In this way, the light-transmitting member 40 block few light beams. When the light emitter 21 emits the light beams, and the light receiver 22 receives the light beams via the light-transmitting member 40, there are the few light beam losses, and the measurement accuracy of the optical heart rate module 20 is high.

In some implementations, a lipid, alcohol, or ketone substance is not selected as a material of the light-transmitting member 40. For example, polypropylene may be used for the light-transmitting member 40. Lipid, alcohol, and ketone substances have short service lives under conditions of a high temperature, high humidity, and soaking in acidic and alkaline sweat. When the lipid, alcohol, or ketone substance is not selected as the material of the light-transmitting member 40, the light-transmitting member 40 still has a long service life in environments of the high temperature, the high humidity, and the acidic and alkaline sweat.

In some implementations, after the light-transmitting member 40 is soaked, an elastic modulus of the light-transmitting member 40 changes by no more than 2% compared with that before the light-transmitting member 40 is soaked. In this way, aging can be avoided when the light-transmitting member 40 is in the environment of the acidic and alkaline sweat for long time, and a gap between the light-transmitting member 40 and the enclosure 10 can be avoided. This reduces a probability that the external impurity such as the water vapor or the dust enters the accommodation space 50 through the gap between the light-transmitting member 40 and the enclosure 10.

In some implementations, the elastic modulus of the light-transmitting member 40 changes by no more than 2% at a temperature of -30°C and at a temperature of +30°C. In this way, risks that the light-transmitting member 40 ages in an environment in which switching is performed between extreme temperatures, and that the gap appears between the light-transmitting member 40 and the enclosure 10 can be reduced. This further reduces the probability that the external impurity such as the water vapor or the dust enters the accommodation space 50 through the gap between the light-transmitting member 40 and the enclosure 10.

In some implementations, the elastic modulus of the light-transmitting member 40 changes by no more than 2% over 5 years. In this way, a service life of the light-transmitting member 40 is long, and a service life of the health monitoring module 100 is long.

The foregoing describes several structures of the health monitoring module 100. The following describes a specific implementation in which the health monitoring module 100 is mounted on the electronic device 1000 and an implementation of signal transmission between the health monitoring module 100 and the mainboard of the electronic device 1000.

As shown in FIG. 1 and FIG. 5, when the health monitoring module 100 is mounted on the electronic device 1000, a mounting hole 2202 may be disposed on the housing 200. The health monitoring module 100 may be electrically connected, through the mounting hole 2202, to the mainboard disposed inside the housing 200.

For example, the flexible printed circuit board 99 may be located outside the accommodation space 50. The flexible printed circuit board 99 may be connected to the circuit board 30, and is electrically connected to the circuit board 30. For example, when the health monitoring module 100 is mounted on the middle frame 220, a part of the flexible printed circuit board 99 may be located in the internal space 1003 of the housing 200, and is configured to connect to the mainboard; a part of the flexible printed circuit board 99 may be located in the mounting hole 2202; and a part of the flexible printed circuit board 99 may be located outside the housing 200, and is configured to electrically connect to the circuit board 30. The circuit board 30 may be electrically connected to the mainboard via the flexible printed circuit board 99. For example, when the health monitoring module 100 is mounted on the middle frame 220 of the electronic device 1000, the mounting hole 2202 may be located on the middle frame 220.

As shown in FIG. 1 and FIG. 4, the flexible printed circuit board 99 may include an electrical connection portion 991, and the electrical connection portion 991 may be configured to electrically connect to the mainboard. For example, a plurality of soldering pads may be disposed on the electrical connection portion 991, and the soldering pads may be configured to electrically connect to the mainboard. When the mainboard is disposed in parallel to the screen 210, the electrical connection portion 991 may also be parallel to the screen 210, to implement the electrical connection between the screen 210 and the mainboard. When the mainboard is disposed perpendicular to a direction of the screen 210, the electrical connection portion may also be disposed perpendicular to the direction of the screen 210, to implement the electrical connection to the mainboard. It may be understood that a manner of disposing the electrical connection portion may be set based on a direction of the mainboard of the electronic device 1000. This is not limited in this application.

In some implementations, the flexible printed circuit board 99 may be electrically connected to the mainboard via a board to board connector (Board to Board Connector, BTB), a hotbar (hotbar) soldering, a zero insertion force connector (Zero Insertion Force connector, ZIF), or the like.

In some implementations, the flexible printed circuit board 99 and the circuit board 30 may be an integrally formed mechanical part. For example, the flexible printed circuit board 99 and the circuit board 30 may be a part of an integrally formed rigid-flex printed circuit board. Alternatively, the flexible printed circuit board 99 and the circuit board 30 may be a part of an integrally formed flexible printed circuit board. In another implementation, the flexible printed circuit board 99 may alternatively be electrically conductive to the circuit board 30 by disposing an electrical connection structure (for example, the BTB connector or the ZIF connector).

For example, a collection path of an electrocardiographic signal of the user may include: After the conductive portion 1 of the enclosure 10 collects the electrical signal of the user, the electrical signal may be transmitted to the mainboard of the electronic device 1000 sequentially via the conductive portion 1, the circuit board 30, and the flexible printed circuit board 99.

For example, the electronic device 1000 may include an electrocardiogram (Electrocardiogram, ECG) chip (not shown in the figure). The ECG chip may be located in the internal space of the housing 200. For example, the ECG chip may be fastened to the mainboard, and is electrically connected to the mainboard. The ECG chip may be electrically connected to the conductive portion 1 of the enclosure 10 via the mainboard, the flexible printed circuit board 99, and the circuit board 30. The ECG chip may be configured to process the electrical signal obtained by the enclosure 10, to obtain the electrocardiogram waveform of the user.

In another implementation, the electronic device 1000 may further include an electrocardiogram chip configured to process a type of electrocardiographic signal other than the ECG waveform. The electrocardiogram chip may be configured to process the electrical signal collected by the conductive portion 1, to obtain electrocardiogram information of the user.

For example, a collection path of a heart rate signal of the user may include: The light beams emitted by the light emitter 21 may irradiate the finger of the user through the light-transmitting hole 11. Then, the light beams may be reflected or scattered by the blood vessels of the finger of the user, and return to the accommodation space 50 through the light-transmitting hole 11. The light beams are received by the light receiver 22. After collecting an optical signal reflected by the tissue of the user, the light receiver 22 of the optical heart rate module 20 converts the optical signal into the electrical signal, and transmits the electrical signal to the mainboard of the electronic device 1000 sequentially via the circuit board 30 and the flexible printed circuit board 99.

For example, the electronic device 1000 may include a PPG chip (not shown in the figure). The PPG chip may be fastened to the mainboard, and is electrically connected to the mainboard. The PPG chip may be electrically connected to the light receiver 22 via the main board and the circuit board 30. The PPG chip may be configured to process the electrical signal collected by the light receiver 22, to obtain a photoplethysmogram (photoplethysmogram, PPG) signal of the user.

In another implementation, the electronic device 1000 may further include a heart rate chip configured to process the heart rate signal by using a method other than a method for processing the PPG signal. The heart rate chip may be configured to process the electrical signal converted by the light receiver 22, to obtain heart rate information of the user.

In some implementations, the PPG signal may be further combined with the foregoing ECG waveform, and more accurate blood pressure of the user and other biological feature information related to the human cardiovascular system are obtained according to an algorithm. For example, the health monitoring module 100 may collect both the ECG waveform and the PPG signal of the user, calculate a time difference PPT between the ECG waveform and the PPG signal according to an algorithm, and obtain blood pressure data of the user through conversion according to a formula.

In some implementations, the electronic device 1000 may include a sealing member 98. The sealing member 98 may be fastened between the flexible printed circuit board 99 and a hole wall of the mounting hole 2202, and is filled in the mounting hole 2202. The sealing member 98 may be configured to seal a gap between the flexible printed circuit board 99 and the mounting hole 2202. In this way, this can prevent the water vapor or the dust from interfering with operation of a component (for example, the mainboard) in the internal space 1003 of the electronic device 1000 by entering the internal space 1003 of the electronic device 1000 through the mounting hole 2202.

In some implementations, the sealing member 98 may be formed after glue is cured. For example, the sealing member 98 may be formed after a waterproof adhesive is cured. In this way, when the electronic device 1000 is in a humid environment or underwater, the sealing member 98 may be used for waterproofing of the electronic device 1000, to prevent the water vapor from entering the electronic device through the mounting hole 2202. The electronic device 1000 may have waterproofing performance.

In some implementations, when the health monitoring module 100 is used as a button of the electronic device 1000, whether the user is in contact with the enclosure 10 may be determined based on whether the PPG signal of the user is collected. In addition, whether the user is in contact with the enclosure 10 may be further determined based on the electrical signal collected by the enclosure 10.

It may be understood that the enclosure 10 of the health monitoring module 100 in this application may be configured to: collect the electrical signal of the user, and obtain an ECG signal. The optical heart rate module 20 may be configured to obtain the PPG signal of the user. After the ECG signal and the PPG signal are processed according to corresponding algorithms, a plurality of physiological indicators of the user such as the electrocardiogram, the heart rate, and the blood pressure can be obtained. In a conventional technical solution, a PPG collection apparatus and an ECG collection apparatus are separately disposed. Two fingers of the user need to separately be in contact with the PPG collection apparatus and the ECG collection apparatus, to complete collection of the ECG signal and the PPG signal. When the health monitoring module 100 in this application collects the ECG signal and the PPG signal of the user, the user only needs to place one finger on the top face 12 of the enclosure 10, so that an operation is more convenient.

The health monitoring module 100 integrates the button function and the health monitoring function. In comparison with a solution in which a button function module and a health monitoring function module are separately disposed, the health monitoring module 100 in this application achieves multifunctionality and has a small size. When the health monitoring module 100 is mounted on the electronic device 1000, miniaturization of the electronic device 1000 is facilitated.

In addition, the enclosure 10 and the optical heart rate module 20 may be stacked in the thickness direction (the Z-axis direction) of the health monitoring module 100. The health monitoring module 100 may occupy a small surface of the electronic device 1000. This helps save stacking space of the entire device. More other functional components may be disposed on a surface of the electronic device 1000, to meet diversified requirements of the user.

In addition, in this application, a blood pressure signal of the user is measured based on the combination of the ECG waveform and the PPG signal. In comparison with a conventional product such as a sphygmomanometer, no mechanisms such as an air pump and a bladder need to be disposed, and a size is smaller. When the health monitoring module 100 in this application measures the blood pressure, the user only needs to place the finger on the enclosure 10 to complete blood pressure monitoring by one tap. A monitoring step is simple, facilitating the user.

In another implementation, a temperature sensor may further be disposed on the top face 12 of the enclosure 10 for the health monitoring module 100. When the user touches the enclosure 10, a temperature change at a position of the top face 12 of the enclosure 10 can be identified by the temperature sensor, to determine whether the user touches the top face 12 of the enclosure 10.

FIG. 8 is a diagram of a structure of a conductive member 60 shown in FIG. 6 in an implementation.

As shown in FIG. 5 and FIG. 8, the health monitoring module 100 may further include the conductive member 60. The conductive member 60 may be located in the accommodation space 50. The conductive member 60 and the optical heart rate module 20 (the light emitter 21 and the light receiver 22) may be spaced. The conductive member 60 may be electrically connected between the conductive portion 1 and the circuit board 30, to transmit the electrical signal collected by the conductive portion 1 to the circuit board 30.

It may be understood that, when the health monitoring module 100 may further include the conductive member 60, a collection path of the ECG waveform of the user may include: After the conductive portion 1 of the enclosure 10 collects the electrical signal of the user, the electrical signal may be transmitted to the mainboard of the electronic device 1000 sequentially via the conductive portion 1, the conductive member 60, the circuit board 30, and the flexible printed circuit board 99.

In some implementations, the conductive member 60 may be elastic. The conductive member 60 may abut against the conductive portion 1 of the enclosure 10 and the circuit board 30, and is electrically connected to the conductive portion 1 and the circuit board 30. It may be understood that, in comparison with a non-elastic conductive structure 64 such as a metal trace or a flexible printed circuit board, the conductive member 60 is elastic, the conductive member 60 abuts against the conductive portion 1 of the enclosure 10 and the circuit board 30, and the conductive member 60 does not easily shift, so that reliability of the electrical connection between the conductive member 60 and the conductive portion 1, and reliability of the electrical connection between the conductive member 60 and the circuit board 30 can be improved.

As shown in FIG. 6 and FIG. 8, the conductive member 60 may include a first surface 61 and a second surface 62 that are disposed back to back. A part of region of the first surface 61 abuts against the circuit board 30, another part of region of the first surface 61 is recessed in a direction away from the circuit board 30 and is spaced apart from the circuit board 30. The second surface 62 abuts against the conductive portion 1 of the enclosure 10. In this way, first space is formed between the another part of region of the first surface 61 and the circuit board 30. When the conductive member 60 abuts against the conductive portion 1 of the enclosure 10 and the circuit board 30, the first space may provide deformation space.

In some implementations, a hole 63 may be disposed on the conductive member 60. In this way, when the conductive member 60 abuts against the conductive portion 1 of the enclosure 10 and the circuit board 30, the hole 63 may provide deformation space. There may be one or more holes 63. FIG. 8 shows two holes 63.

In some implementations, the conductive member 60 may include the conductive structure 64 and an elastic structure 65. The conductive structure 64 may wrap the elastic structure 65. The conductive structure 64 is configured to electrically connect the conductive portion 1 to the circuit board 30. The elastic structure 65 is configured to provide elasticity. When the conductive member 60 abuts against the circuit board 30 and the conductive portion 1, the elastic structure 65 is compressed, and the conductive structure 64 is subject to an elastic force that is of the elastic structure 65 and that is for restoring deformation. A part of the conductive member 60 abuts against the elastic structure 65 and the circuit board 30, and is electrically connected to the circuit board 30. Another part of the conductive member 60 abuts against the elastic structure 65 and the conductive portion 1 of the enclosure 10, and is electrically connected to the conductive portion 1.

In some implementations, the conductive structure 64 may be made of a conductive metal material such as copper. The elastic structure 65 may be made of an elastic material such as silicone or rubber.

In another implementation, the conductive member 60 may alternatively be entirely made of conductive silicone having a conductive capability. In this way, the conductive member 60 features elasticity of the silicone, and can implement the electrical connection between the circuit board 30 and the conductive portion 1.

In another embodiment, the conductive member 60 may alternatively be of a solid structure. The conductive member 60 may be disposed between the light emitter 21 and the light receiver 22. The conductive member 60 may be used as a light-shielding structure, and is configured to block the light beams emitted by the light emitter 21, to prevent the light beams emitted by the light emitter 21 from interfering with the light receiver 22 receiving external light beams.

In another implementation, a structure such as a conductive spring, a conductive elastomer, or a conductive wire may be further used for the conductive member 60.

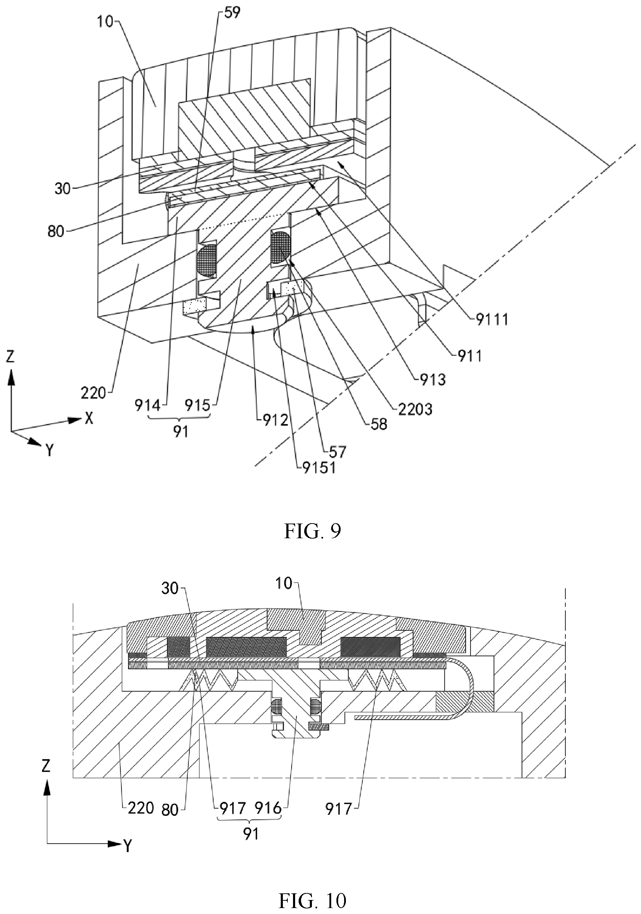

FIG. 9 is a partial sectional view of the electronic device 1000 shown in FIG. 1 along a section line C-C in an implementation.

As shown in FIG. 4, FIG. 5, and FIG. 9, the health monitoring module 100 may further include a pressure sensing circuit 80 and a pressure sensing assembly 90, the pressure sensing assembly 90 may be fastened to a side that is of the circuit board 30 and that faces away from the enclosure 10, and the pressure sensing circuit 80 may be fastened to the pressure sensing assembly 90. When the enclosure 10 is subject to pressure, and the pressure sensing assembly 90 may be deformed, the pressure sensing circuit 80 may be configured to detect the deformation of the pressure sensing assembly 90.

For example, a resistance value of the pressure sensing circuit 80 may change based on a deformation degree of the pressure sensing assembly 90. A pressure sensing chip (not shown in the figure) may be disposed on the electronic device 1000. For example, the pressure sensing chip may be mounted on the mainboard, and is electrically connected to the mainboard. The pressure sensing chip may be electrically connected to the pressure sensing circuit 80. The electronic device 1000 may provide a current of a fixed voltage value for the pressure sensing circuit 80. When the resistance value of the pressure sensing circuit 80 changes, the current on the pressure sensing circuit 80 also changes accordingly. The pressure sensing chip may determine, based on a magnitude of the current, a magnitude of a force at which the user presses the enclosure 10.

For example, when the user measures the ECG signal and the PPG signal, the user places the finger on the enclosure 10, and the pressure sensing assembly 90 may be deformed to different degrees based on an action force between the finger of the user and the enclosure 10. The pressure sensing circuit 80 may be configured to detect the deformation of the pressure sensing assembly 90. In this way, a deformation degree, of the pressure sensing assembly 90, detected by the pressure sensing circuit 80 may be used to obtain the magnitude of the force at which the finger of the user presses the enclosure 10.

It may be understood that, when the finger of the user is placed at an incorrect position on the enclosure 10, or when the finger is placed at a position that is offset from an ideal monitoring position, the pressure sensing assembly 90 is subject to a small force. The pressure sensing chip may determine, based on a force exerted on the pressure sensing assembly 90, whether the finger of the user is placed in place. In operating processes of the pressure sensing circuit 80 and the pressure sensing assembly 90, a first threshold may be set. The first threshold is a minimum force exerted on the pressure sensing assembly 90 when the finger of the user is placed in place. When the pressure sensing chip detects that a real-time exerted force detected by the pressure sensing assembly 90 is greater than or equal to the first threshold, the pressure sensing chip may control the health monitoring module 100 to start to collect both the ECG signal and the PPG signal of the user, calculate a time difference between the ECG signal and the PPG signal, that is, pulse transit time (Pulse Transit Time, PTT), and finally obtain the blood pressure data of the user through conversion according to a series of formulas.

In some implementations, when the action force between the finger of the user and the enclosure 10 is small, depths of tissue penetrated, beneath the skin of the user, by the light beams emitted by the light emitter 21 are limited, and the blood vessels of the user are at specific depths beneath the skin surface of the user. Generally, a larger value of the force of the user exerting on the enclosure 10 indicates better fitting effect between the finger and a knob 10, and greater depths to which the light beams emitted by the light emitter 21 penetrate the skin of the user. Therefore, when the health monitoring module 100 is configured to measure the PPG signal, a second threshold may be set. When the action force between the finger of the user and the enclosure 10 is greater than or equal to the second threshold, the light beams emitted by the light emitter 21 penetrate the skin of the user to great depths, so that most of the light beams can irradiate positions of the blood vessels beneath the skin of the user. When the pressure sensing chip detects that the real-time exerted force detected by the pressure sensing assembly 90 is greater than or equal to the second threshold, the pressure sensing chip controls the health monitoring module 100 to start to collect the PPG signal of the user. In this way, accuracy of the collected PPG signal is good.

In addition, when the real-time exerted force detected by the pressure sensing assembly 90 is greater than or equal to the second threshold, the pressure sensing chip controls the health monitoring module 100 to start to collect the ECG signal of the user, so that the action force between the user and the enclosure 10 is within a large range, and reliability of the electrical connection between the enclosure 10 and the user is good. In this way, accuracy of the collected ECG signal is good.

For example, the first threshold and the second threshold may be equal or may not be equal.

In some implementations, the pressure sensing circuit 80 and the pressure sensing assembly 90 may be configured to measure a water depth. It may be understood that when the user is underwater, water pressure can also cause the pressure sensing assembly 90 to deform, so that the pressure sensing circuit 80 can detect a deformation degree of the pressure sensing assembly 90. The pressure sensing chip can detect a value of the water pressure based on an electrical signal of the pressure sensing circuit 80. The water pressure is proportional to the water depth. Real-time underwater depth information of the user may be obtained through calculation according to a formula, so that the health monitoring module 100 can have an underwater depth detection function.

It may be understood that, in comparison with a conventional technical solution in which a depth meter is disposed to measure a water depth, the depth meter needs an opening to transfer water pressure. In this application, the health monitoring module 100 directly transfers the water pressure to the pressure sensing assembly 90 via the enclosure 10, and obtains the water depth through calculation according to the formula. The health monitoring module 100 in this application can measure the water depth without the additionally disposed depth meter component, has a smaller size, and is more portable. In addition, the water pressure is directly transferred to the pressure sensing assembly 90 via the top face 12 of the enclosure 10, and no opening is needed. The top face 12 of the enclosure 10 is the externally exposed structure and is easy to clean, and the health monitoring module 100 is not prone to a contamination or clogging problem.

It may be understood that detection accuracy of the pressure sensor may be customized according to an accuracy requirement and a depth measurement range requirement. For example, accuracy and a range of pressure detection is improved by increasing an area of a strain gauge of the pressure sensor.

For example, the accuracy of the pressure detection of the health monitoring module 100 may reach 5 gram-force (gF) (which may be equivalent to pressure at a water depth of 0.15 meters), and the measurement range of the pressure detection may reach 1500 gF (which may be equivalent to pressure at a water depth of 50 meters).

In some implementations, the electronic device 1000 may further include a motor (not shown in the figure). The motor may be mounted on the housing 200. For example, the motor may be disposed in the internal space of the electronic device 1000, and the health monitoring module 100 may be electrically connected to the motor. The motor may be configured to vibrate based on data monitored by the health monitoring module 100. The following specifically describes, with reference to the accompanying drawings, several implementations in which the motor vibrates based on the data monitored by the health monitoring module 100.

In some implementations, when the health monitoring module 100 is used as the touch-sensitive button of the electronic device 1000, the motor may vibrate to prompt the user whether an operation on the button is effective.

In some implementations, the pressure sensing circuit 80 may be electrically connected to the motor of the electronic device 1000. When the user presses the enclosure 10, and the pressure sensing circuit 80 detects that the pressure sensing assembly 90 is deformed, the motor may vibrate. The pressure sensing circuit 80 and the pressure sensing assembly 90 may cooperate with the motor to implement the virtual button function of the health monitoring module 100. For example, a corresponding threshold is set in an algorithm of the entire device. When the user presses the enclosure 10 with the finger, the resistance value of the pressure sensing circuit 80 changes, and the electrical signal is generated. This is calculated by the pressure sensing chip inside the entire device. The pressure sensing chip may be electrically connected to a motor, and control the motor to generate vibration, to implement a function similar to the touch-sensitive button.

In some implementations, a detection result of the pressure sensing circuit 80 may be further used to determine an accidental touch. Accidental touches may include a button accidental touch and a health monitoring accidental touch. For example, when the pressure sensing circuit 80 detects that a force at which the user presses the enclosure 10 is greater than the threshold, it is determined that the user presses the enclosure 10; and when the force is less than the threshold, it is determined to be the accidental touch. It may be understood that a value of the threshold may be adjusted based on an actual use case.

In some implementations, when the pressure sensing circuit identifies that the force exerted on the pressure sensing assembly 90 is less than the first threshold, the pressure sensing circuit controls the motor to vibrate, to prompt the user that the position of the finger placed on the enclosure 10 is offset from the ideal monitoring position.

In some implementations, when the pressure sensing circuit identifies that the force exerted on the pressure sensing assembly 90 is less than the second threshold, the pressure sensing circuit controls the motor to vibrate, to prompt the user to increase the force, thereby improving collection accuracy of the ECG signal and the PPG signal.

In some implementations, when the health monitoring module 100 collects the health parameters of the user, and when the collection of the PPG signal and ECG signal is completed, the motor may vibrate to prompt that monitoring is completed.

In some implementations, the pressure sensing circuit 80 may be fastened to the circuit board 30 through adhesive bonding.

In some implementations, the pressure sensing circuit 80 may be electrically connected to the circuit board 30. The electrical signal of the pressure sensing circuit 80 may be transmitted to the mainboard of the electronic device 1000 via the circuit board 30 and the flexible printed circuit board 99. In this way, when the health monitoring module 100 is mounted on the middle frame 220, only one mounting hole 2202 needs to be disposed on the middle frame 220 for the flexible printed circuit board 99 to pass through, and a structure of the middle frame 220 is simple. In another implementation, the pressure sensing circuit 80 may alternatively be directly electrically connected to the mainboard of the electronic device 1000, and the electrical signal of the pressure sensing circuit 80 does not pass through the circuit board 30 and the flexible printed circuit board 99. In this case, a through hole may be additionally disposed on the middle frame 220 for the electrical signal of the pressure sensing circuit 80 to pass through.

In some implementations, the pressure sensing circuit 80 and the circuit board 30 may be an integrally formed mechanical part. For example, the pressure sensing circuit 80 and the circuit board 30 may be a part of an integrally formed rigid-flex printed circuit board. Alternatively, the pressure sensing circuit 80 and the circuit board 30 may be a part of an integrally formed flexible printed circuit board.

In some implementations, the pressure sensing assembly 90 may include a stem 91. The stem 91 may include a first surface 911 and a second surface 912. The first surface 911 of the stem 91 and the second surface 912 of the stem 91 are disposed back to back. A part of region of the first surface 911 of the stem 91 may be fastened to the side that is of the circuit board 30 and that faces away from the enclosure 10. The pressure sensing circuit 80 may be disposed in another part of region of the first surface 911 of the stem 91. The pressure sensing circuit 80 and the circuit board 30 are spaced apart. When the enclosure 10 is subject to the pressure, the stem 91 is deformed. The pressure sensing circuit 80 may be configured to detect the deformation of the stem 91.

It may be understood that the pressure sensing circuit 80 is mounted on the first surface 911 of the stem 91 rather than another position of the stem 91. The part of region of the first surface 911 of the stem 91 may be fastened to the side that is of the circuit board 30 and that faces away from the enclosure 10. A distance between the pressure sensing circuit 80 and the circuit board 30 may be short. When the pressure sensing circuit 80 is electrically connected to the circuit board 30, a length of an electrical connection structure between the pressure sensing circuit 80 and the circuit board 30 is short, and a size of the electrical connection structure is small. This facilitates the miniaturization of the health monitoring module 100.

In addition, the pressure sensing circuit 80 and the circuit board 30 are spaced apart. Space between the pressure sensing circuit 80 and the circuit board 30 may be used to provide space for the deformation of the stem 91, to prevent the stem 91 from pressing another nearby component when the stem 91 is deformed.

As shown in FIG. 9, a stepped face 913 may be disposed on the stem 91. The stepped face 913 may be connected to the middle frame 220. The stepped face 913 is located on a side that is of the first surface 911 and that is away from the circuit board 30. When the enclosure 10 is subject to the pressure, the enclosure 10 moves downward, and the pressure F1 of the user on the enclosure 10 is transferred to the part of region of the first surface 911 of the stem 91 via the enclosure 10 and the circuit board 30. The stepped face 913 of the stem 91 may abut against the middle frame 220. A reaction force F2 of the middle frame 220 may exert on the stepped face 913 of the stem 91, and a direction of the reaction force F2 is opposite to that of a force of pressing the enclosure 10 by the user. In this way, the another part of region (that is, a region to which the pressure sensing circuit 80 is fastened) of the first surface 911 may be deformed under exertion of F1 and F2, and the pressure sensing circuit 80 may detect the deformation of the part.

In some implementations, a groove 9111 may be disposed on the first surface 911 of the stem 91, and the pressure sensing circuit 80 may be disposed in the groove 9111. For example, the first surface 911 of the stem 91 may be recessed towards a side away from the circuit board 30 to form the groove 9111, and a wall face of the groove 9111 is a part of the first surface 911 of the stem 91. A depth of the groove 9111 may be set to be slightly greater than thickness of the pressure sensing circuit 80, and the pressure sensing circuit 80 is fastened to a bottom face of the groove 9111. In this way, the pressure sensing circuit 80 and the circuit board 30 can be spaced.

In some implementations, the depth of the groove 9111 is within a range of 0.2 mm to 0.5 mm. For example, the depth of the groove 9111 may be 0.2 mm, 0.3 mm, 0.4 mm, or 0.5 mm.

In some implementations, the stem 91 may include a first part 914 and a second part 915 (for ease of understanding, the first part 914 and the second part 915 are schematically divided based on dashed lines in FIG. 9). The first part 914 is plate-shaped, and the second part 915 is rod-shaped. The second part 915 is located on a side that is of the first part 914 and that faces away from the circuit board 30. An end of the second part 915 is connected to a middle of the first part 914. The first surface 911 of the stem 91 is a surface of a side that is of the first part 914 and that faces away from the second part 915. The pressure sensing circuit 80 may be disposed in another part of region of a second face 9142. In this way, the stem 91 may be in a "T" shape. When the enclosure 10 is subject to the pressure, and the first part 914 may be deformed, the pressure sensing circuit 80 is configured to detect the deformation of the first part 914. The second surface 912 of the stem 91 may be a surface of a side that is of the second part 915 and that faces away from the first part 914.

It may be understood that the stem 91 is set to a "T" structure. Two ends of the first part 914 may be fastened to the circuit board 30. Connection positions between the first surface 911 and the circuit board 30 may include a first position P1 and a second position P2 (as shown in FIG. 5). The groove 9111 may be disposed between the first position P1 and the second position P2. When the enclosure 10 is subject to the pressure, the two ends of the first part 914 are subject to two forces F1, and the middle of the first part 914 is subject to a force F2, so that the deformation of the first part 914 can be large, and a resistance change of the pressure sensing circuit 80 is large. This facilitates detection of the pressure applied by the user on the enclosure 10. In addition, when the user presses the enclosure 10, the enclosure 10 is not easy to tilt towards one side.

In another implementation, the first part 914 may alternatively be cylindrical. The groove 9111 may be disposed at the middle of the first surface 911.

As shown in FIG. 9, the health monitoring module 100 may further include a first waterproof member 59, and the first waterproof member 59 may be connected to the first part 914 and covers the pressure sensing circuit 80. In this way, the first waterproof member 59 may be configured to implement waterproofing and dustproofing for the pressure sensing circuit 80, to prevent water vapor or dust from interfering with operation of the pressure sensing circuit 80 through a gap between the circuit board 30 and the stem 91. For example, the first waterproof member 59 may be formed through curing of a waterproof adhesive.

In some implementations, the groove 9111 may be disposed on the first surface 911 of the stem 91, and the pressure sensing circuit 80 may be disposed in the groove 9111. The first waterproof member 59 may be filled in the groove 9111, is connected to the first part 914, and covers the pressure sensing circuit 80.

In some implementations, Shore hardness of the first waterproof member 59 may be in a range greater than or equal to 20° and less than or equal to 30°.

In some implementations, the lipid, alcohol, or ketone substance is not selected as a material of the first waterproof member 59. In this way, the first waterproof member 59 still has a long service life in the environments of the high temperature, the high humidity, and the acidic and alkaline sweat.

In some implementations, after the first waterproof member 59 is soaked, an elastic modulus of the first waterproof member 59 changes by no more than 2% compared with that before the first waterproof member 59 is soaked. In this way, in the environment of the acidic and alkaline sweat for long time, the first waterproof member 59 can age slowly, and a risk of corrosion of the pressure sensing circuit 80 by the impurities such as the water vapor or the dust can be reduced.

In some implementations, the elastic modulus of the first waterproof member 59 changes by no more than 2% at the temperature of -30°C and at the temperature of +30°C. In this way, in the environment in which switching is performed between the extreme temperatures, the first waterproof member 59 can age slowly, and the risk of the corrosion of the pressure sensing circuit 80 by the impurities such as the water vapor or the dust can be reduced.

In some implementations, the elastic modulus of the first waterproof member 59 changes by no more than 2% over 5 years. In this way, a service life of the first waterproof member 59 is long, and the risk of the corrosion of the pressure sensing circuit 80 by the impurities such as the water vapor or the dust is reduced.

In some implementations, the health monitoring module 100 further includes a second waterproof member 58, and the second waterproof member 58 is sleeved on the second part 915. When the health monitoring module 100 is fastened to the middle frame 220 of the electronic device 1000, a fastening hole 2203 may be disposed on the middle frame 220, and a part of the second part 915 is located in the fastening hole 2203. In this case, the second waterproof member 58 may abut against the second part 915 and a wall face of the fastening hole 2203. It may be understood that, the probability that the operation of the component (for example, the mainboard) in the internal space of the electronic device 1000 is interfered with by the external water vapor or dust entering the internal space of the electronic device 1000 through a gap between the second part 915 and the fastening hole 2203 can be reduced by disposing the second waterproof member 58.

In some implementations, the second waterproof member 58 may be made of the elastic material such as the silicone or the rubber.

In some implementations, the health monitoring module 100 may further include a jump ring 57. A clamping slot 9151 may be disposed on the second part 915. An opening of the clamping slot 9151 may be located on a side face of the second part 915. When the health monitoring module 100 is mounted on the middle frame 220, an opening of the clamping slot 9151 may be located in the internal space 1003 of the electronic device 1000. A part of the jump ring 57 may be fastened in the clamping slot 9151, and a part of the jump ring 57 extends out of the clamping slot 9151. In this way, the jump ring 57 can prevent the health monitoring module 100 from being loosened towards the outside of the electronic device 1000.

In another implementation, the health monitoring module 100 may also include a latch, and the latch is fastened to the second part 915. When the health monitoring module 100 is mounted on the middle frame 220, the latch may be located in the internal space 1003 of the electronic device 1000, and a length of the latch may be greater than a hole diameter of the fastening hole 2203. In this way, the latch can also prevent the health monitoring module 100 from becoming loose towards the outside of the electronic device 1000.

It may be understood that, as shown in FIG. 1, FIG. 2, and FIG. 9, the health monitoring module 100 in this application may be mounted on the housing 200 of the electronic device 1000 as an independent product. In a process in which the health monitoring module 100 is mounted on the housing 200, the flexible printed circuit board 99 of the health monitoring module 100 may enter the internal space 1003 of the electronic device 1000 through the mounting hole 2202. The fastening hole 2203 may be disposed on the housing 200, and the pressure sensing assembly 90 can implement limiting relative to the housing 200 via the second waterproof member 58 and the jump ring 57. A probability that the external water vapor or dust enters the internal space of the electronic device 1000 through the gap between the second part 915 and the fastening hole 2203 can be reduced by the second waterproof member 58. The sealing member 98 is filled in the mounting hole 2202. The sealing member 98 may be configured to seal the gap between the flexible printed circuit board 99 and the mounting hole 2202, to prevent the water vapor or the dust from entering the internal space 1003 of the electronic device 1000 through the mounting hole 2202. In this way, assembly and sealing between the health monitoring module 100 and the housing 200 are completed. An overall waterproof function of the electronic device 1000 is good. In some implementations, the electronic device 1000 may reach the waterproof level of 5 ATM.

In some implementations, the sealing member 98 may be formed by applying and curing a waterproof adhesive in the mounting hole 2202. It may be understood that, in comparison with that an elastic material such as a rubber plug is used for the sealing member 98, the waterproof adhesive has mobility before being cured, can be filled in a small gap, can well adapt to a shape of the gap between the mounting hole 2202 and the flexible printed circuit board 99, and can better seal the gap between the mounting hole 2202 and the flexible printed circuit board 99.

The foregoing describes a specific implementation of the pressure sensing circuit 80 and the pressure sensing assembly 90. The following further describes several implementations of the pressure sensing circuit 80 and the pressure sensing assembly 90 with reference to the accompanying drawings.

In some implementations, technical content that is the same as that of the health monitoring module 100 in the foregoing implementations is not described again. FIG. 10 is a partial sectional view of the electronic device 1000 shown in FIG. 1 along a section line A-A in still another implementation.

As shown in FIG. 10, the stem 91 may include a fastening portion 916 and a deformation portion 917. The fastening portion 916 may be fastened to the side that is of the circuit board 30 and that faces away from the enclosure 10. One end of the deformation portion 917 is fastened to the fastening portion 916, and the other end of the deformation portion 917 may be connected to the middle frame 220. The pressure sensing circuit 80 is fastened to the deformation portion 917. When the user presses the enclosure 10, the fastening portion 916 may move downward, the deformation portion 917 abuts against the circuit board 30 and the middle frame 220, and the deformation portion 917 is deformed. The pressure sensing circuit 80 may be fastened to the above of the deformation portion 917. The pressure sensing circuit 80 may be configured to detect the deformation of the deformation portion 917.

In some implementations, when the user presses the enclosure 10, the deformation portion 917 abuts against the circuit board 30 and the middle frame 220. The deformation portion 917 is deformed in the thickness direction of the health monitoring module 100 under an abutting force in the thickness direction.

In some implementations, there may be two deformation portions 917, and the two deformation portions 917 are respectively fastened to two ends of the fastening portion 916. A quantity of pressure sensing circuits 80 may alternatively be set to two, and the two pressure sensing circuits 80 and the two deformation portions 917 are disposed in a one-to-one correspondence.

As shown in FIG. 10, a first deformation portion 917, a fastening portion 916, and a second deformation portion 917 are sequentially disposed in the stem 91 in the Y-axis direction from left to right. The circuit board 30, the deformation portions 917, and a part of the middle frame 220 are sequentially disposed from top to bottom in the Z-axis direction.

In some implementations, the deformation portion 917 may be in a continuous "W" shape, or may be in a continuous "S" shape, a continuous "V" shape, or the like. A shape of the deformation portion 917 may be designed based on a requirement. This is not limited in this application.

In some implementations, the deformation portion 917 may be a leaf spring.

In another implementation, there may alternatively be one deformation portion 917.

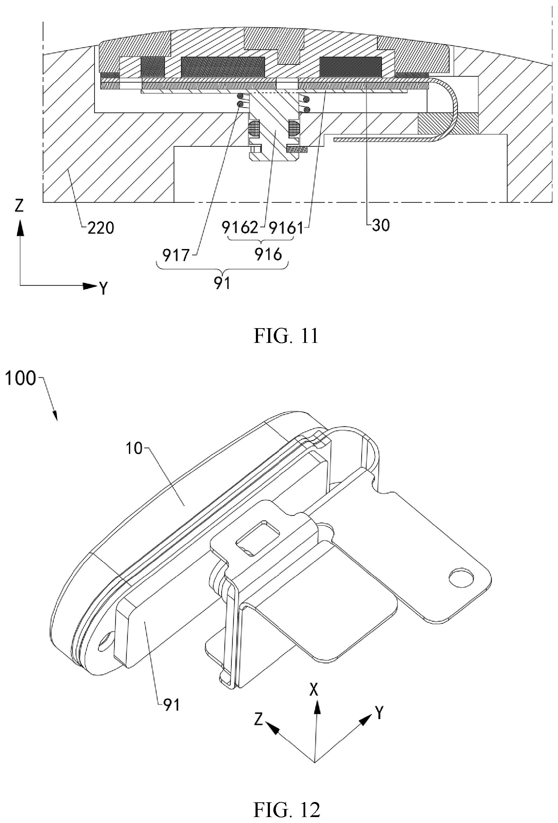

In some implementations, technical content that is the same as that of the health monitoring module 100 in the foregoing implementations is not described again. FIG. 11 is a partial sectional view of the electronic device 1000 shown in FIG. 1 along a section line A-A in yet another implementation.

As shown in FIG. 11, the stem 91 may include a fastening portion 916 and a deformation portion 917. The fastening portion 916 may include a first part 9161 and a second part 9162. The first part 9161 may be plate-shaped, and the second part 9162 may be rod-shaped. The first part 9161 of the fastening portion 916 may be fastened to the side that is of the circuit board 30 and that faces away from the enclosure 10. The second part 9162 may be located on a side that is of the first part 9161 and that faces away from the circuit board 30, and one end of the second part 9162 is connected to a middle of the first part 9161. The deformation portion 917 may be of a spiral structure, and is sleeved on the second part 9162. When the user presses the enclosure 10, the fastening portion 916 may move downward, one end of the deformation portion 917 presses against the first part 9161 of the fastening portion 916, the other end of the fastening portion 916 presses against the middle frame 220, and the deformation portion 917 is deformed. The pressure sensing circuit 80 may be configured to detect the deformation of the deformation portion 917.

As shown in FIG. 11, the first part 9161 and the second part 9162 of the fastening portion 916 are sequentially disposed from top to bottom in the Z-axis direction. The circuit board 30, the first part 9161, the deformation portions 917, and a part of the middle frame 220 are sequentially disposed from top to bottom in the Z-axis direction.

In some implementations, the deformation portion 917 may be a spring.

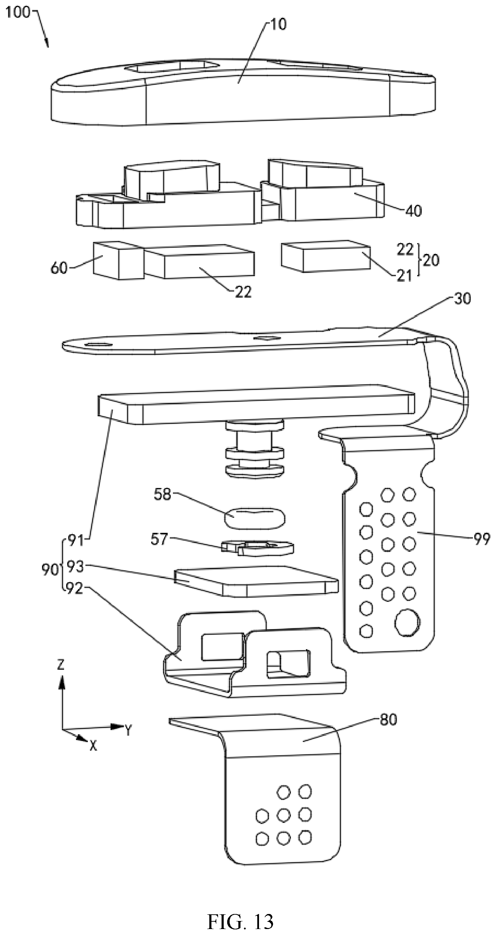

In some implementations, technical content that is the same as that of the health monitoring module 100 in the foregoing implementations is not described again. FIG. 12 is a diagram of a structure of the health monitoring module 100 shown in FIG. 1 in another implementation. FIG. 13 is an exploded diagram of the health monitoring module 100 shown in FIG. 12 in an implementation.

As shown in FIG. 12 and FIG. 13, the health monitoring module 100 may include the enclosure 10, the optical heart rate module 20, the circuit board 30, the light-transmitting member 40, the jump ring 57, the second waterproof member 58, the conductive member 60, the flexible printed circuit board 99, the pressure sensing circuit 80, and the pressure sensing assembly 90. For a manner of disposing the enclosure 10, the optical heart rate module 20, the circuit board 30, the light-transmitting member 40, the jump ring 57, the second waterproof member 58, the conductive member 60, and the flexible printed circuit board 99, refer to the manner of disposing the enclosure 10, the optical heart rate module 20, the circuit board 30, the light-transmitting member 40, the jump ring 57, the second waterproof member 58, the conductive member 60, and the flexible printed circuit board 99 in the foregoing embodiments. Details are not described herein again.

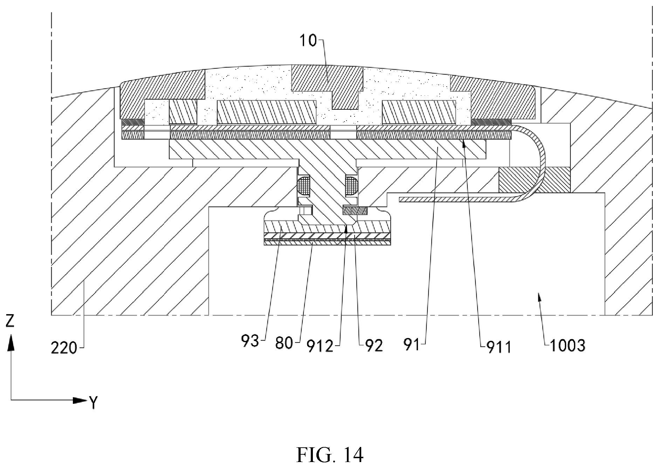

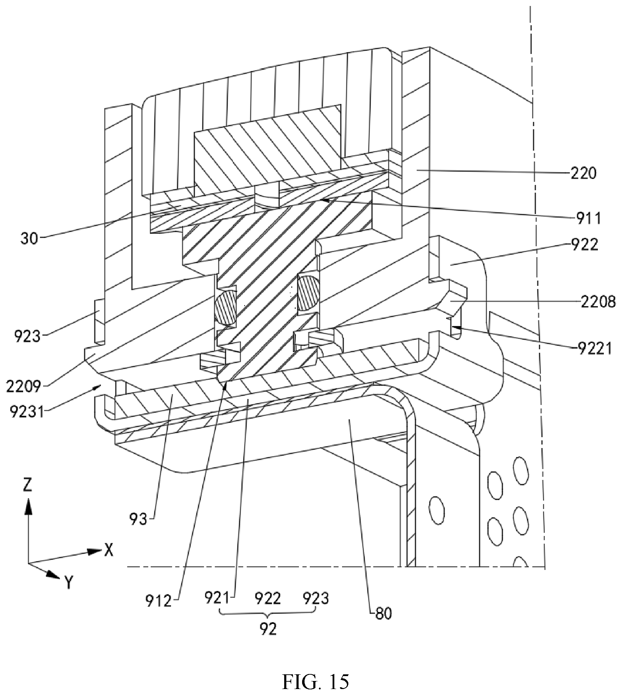

FIG. 14 is a partial sectional view of the electronic device 1000 shown in FIG. 1 along a section line A-A in yet another implementation. FIG. 15 is a partial sectional view of the electronic device 1000 shown in FIG. 1 along a section line C-C in another implementation. It may be understood that FIG. 14 and FIG. 15 are intended to illustrate assembly of the health monitoring module 100 in FIG. 12 on the electronic device 1000.

As shown in FIG. 13 to FIG. 15, the pressure sensing assembly 90 may include the stem 91 and a support steel sheet 92. The stem 91 may include the first surface 911 and the second surface 912. The first surface 911 of the stem 91 and the second surface 912 of the stem 91 are disposed back to back. The first surface 911 of the stem 91 may be fastened to the side that is of the circuit board 30 and that faces away from the enclosure 10. The support steel sheet 92 is connected to the second surface 912 of the stem 91, and the pressure sensing circuit 80 is fastened to a side that is of the support steel sheet 92 and that is away from the stem 91. When the enclosure 10 is subject to the pressure, the support steel sheet 92 may be elastically deformed, and the pressure sensing circuit 80 may be configured to detect the deformation of the support steel sheet 92.

It may be understood that the pressure sensing circuit 80 and the circuit board 30 are spaced apart. When the pressure sensing circuit 80 is faulty, the pressure sensing circuit 80 may be separately repaired, and the optical heart rate module 20 is not affected, so that the repair is more convenient.

In some implementations, when the health monitoring module 100 is mounted on the middle frame 220, a part of the stem 91 is located in the internal space of the electronic device 1000, and the other part of the stem 91 protrudes from the middle frame 220. The support steel sheet 92 and the pressure sensing circuit 80 may be mounted in the internal space of the electronic device 1000. In this way, in comparison with the foregoing embodiment (as shown in FIG. 5), the support steel sheet 92 and the pressure sensing circuit 80 may be mounted in the internal space 1003 of the electronic device 1000. When the electronic device 1000 falls or is hit by a large external force, the middle frame 220 can protect the pressure sensing circuit 80 and the support steel sheet 92, and reliability of the electronic device 1000 is good. In addition, a waterproof structure does not need to be additionally disposed on the health monitoring module 100 to perform waterproofing on the pressure sensing circuit 80.

In some implementations, the support steel sheet 92 may include a main body portion 921, a first ear portion 922, and a second ear portion 923. The first ear portion 922 and the second ear portion 923 are respectively connected to two ends of the main body portion 921. When the health monitoring module 100 is mounted on the middle frame 220, the first ear portion 922 and the second ear portion 923 may be fastened to the middle frame 220. For example, the middle frame 220 may include a first convex portion 2208 and a second convex portion 2209. A fifth through hole 9221 may be disposed on the first ear portion 922, and a sixth through hole 9231 may be disposed on the second ear portion 923. When the health monitoring module 100 is mounted on the middle frame 220, the first convex portion 2208 may be partially located in the fifth through hole 9221; and the second convex portion 2209 may be partially located in the sixth through hole 9231. In this way, when the enclosure 10 is subject to the pressure, the stem 91 moves, relative to the fastening hole 2203, towards the internal space of the electronic device 1000, the stem 91 can abut against the main body portion 921 of the support steel sheet 92, and the main body portion 921 is deformed due to a force. The pressure sensing circuit 80 is configured to detect the deformation of the support steel sheet 92.

In some implementations, the pressure sensing assembly 90 may further include an elastic member 93. The elastic member 93 abuts against the stem 91 and the support steel sheet 92, and the elastic member 93 is elastic. It may be understood that, by disposing the elastic member 93, structural tolerances of the stem 91 and the support steel sheet 92 can be accommodated, and assembly tolerances between the stem 91 and the support steel sheet 92 can be accommodated, to avoid that an excessively large abutting force between the stem 91 and the support steel sheet 92 causes large deformation of the support steel sheet 92, leading to reduction in pressure sensing detection accuracy or a pressure sensing detection failure. In addition, when the health monitoring module 100 is used as a button, and the user presses the health monitoring module 100, the elastic member 93 may be used for cushioning, and the elastic member 93 may further improve a tactile feeling for use.

As shown in FIG. 13 to FIG. 15, the circuit board 30, the stem 91, the elastic member 93, a part of the support steel sheet 92, and the pressure sensing circuit 80 are sequentially disposed from top to bottom in the Z-axis direction.

In some implementations, technical content that is the same as that of the health monitoring module 100 in the foregoing implementations is not described again.

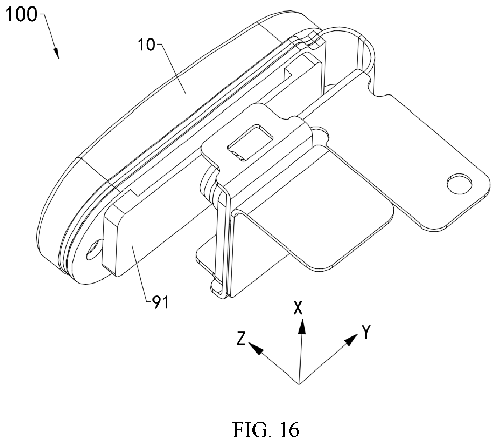

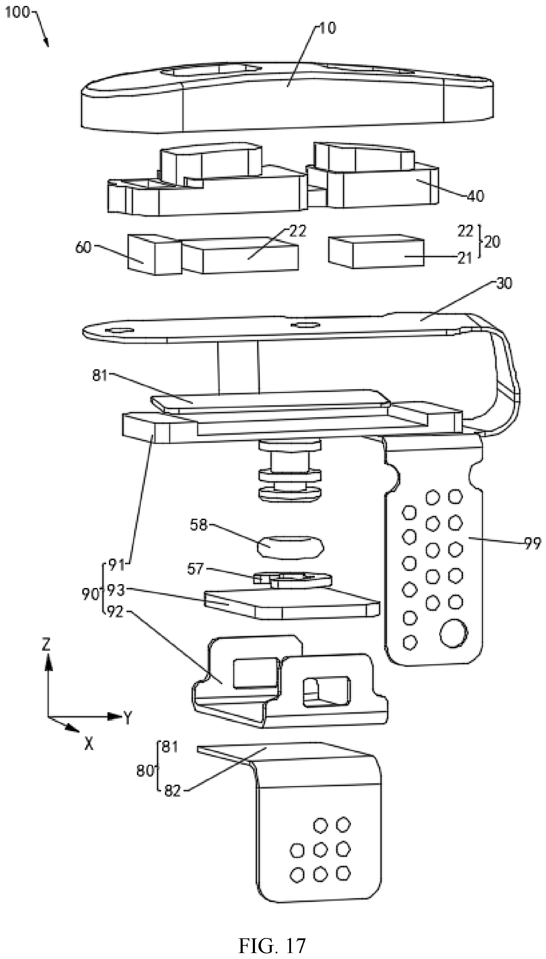

FIG. 16 is a diagram of a structure of the health monitoring module 100 shown in FIG. 1 in another implementation. FIG. 17 is an exploded diagram of the health monitoring module 100 shown in FIG. 16 in an implementation.

As shown in FIG. 16 and FIG. 17, the health monitoring module 100 may include the enclosure 10, the optical heart rate module 20, the circuit board 30, the light-transmitting member 40, the jump ring 57, the second waterproof member 58, the conductive member 60, the flexible printed circuit board 99, the pressure sensing circuit 80, and the pressure sensing assembly 90. For a manner of disposing the enclosure 10, the optical heart rate module 20, the circuit board 30, the light-transmitting member 40, the jump ring 57, the second waterproof member 58, the conductive member 60, and the flexible printed circuit board 99, refer to the manner of disposing the enclosure 10, the optical heart rate module 20, the circuit board 30, the light-transmitting member 40, the jump ring 57, the second waterproof member 58, the conductive member 60, and the flexible printed circuit board 99 in the foregoing embodiments. Details are not described herein again.

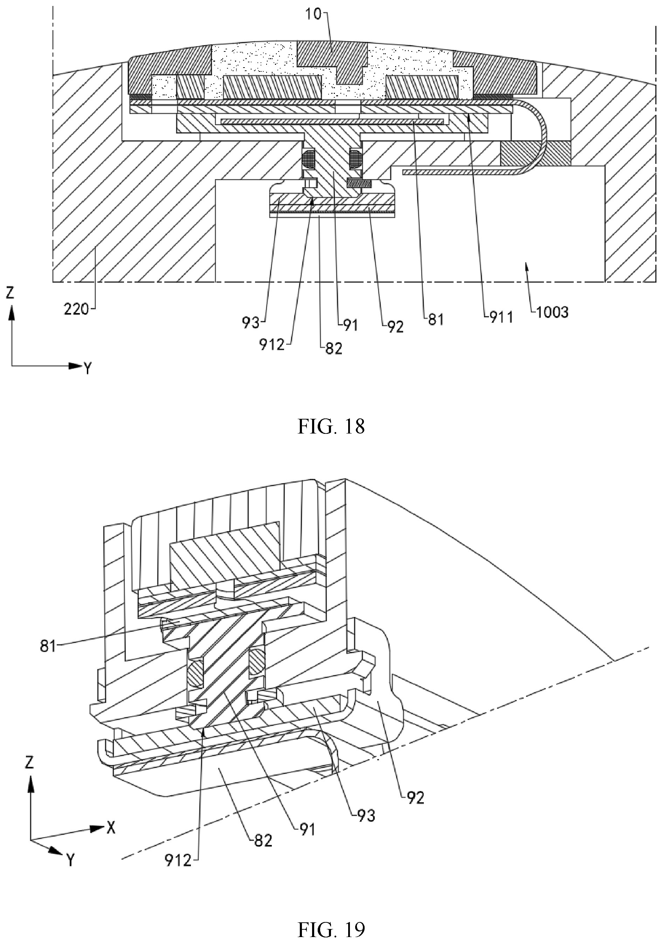

FIG. 18 is a partial sectional view of the electronic device 1000 shown in FIG. 1 along a section line A-A in yet another implementation. FIG. 19 is a partial sectional view of the electronic device 1000 shown in FIG. 1 along a section line C-C in still another implementation. It may be understood that FIG. 18 and FIG. 19 are intended to illustrate assembly of the health monitoring module 100 in FIG. 16 on the electronic device 1000.

As shown in FIG. 17 to FIG. 19, the pressure sensing assembly 90 may include the stem 91, a support steel sheet 92, and an elastic member 93. The stem 91 may include the first surface 911 and the second surface 912. The first surface 911 of the stem 91 and the second surface 912 of the stem 91 are disposed back to back. The part of region of the first surface 911 of the stem 91 is fastened to the side that is of the circuit board 30 and that faces away from the enclosure 10, and the support steel sheet 92 is connected to the second surface 912 of the stem 91.

The pressure sensing circuit 80 may include a first pressure sensing circuit 81 and a second pressure sensing circuit 82, the first pressure sensing circuit 81 is disposed in another part of region of the first surface 911 of the stem 91, and the first pressure sensing circuit 81 and the circuit board 30 are spaced apart. The second pressure sensing circuit 82 is fastened to a side that is of the support steel sheet 92 and that is away from the stem 91. When the enclosure 10 is subject to the pressure, the stem 91 is deformed, and the support steel sheet 92 is deformed, the first pressure sensing circuit 81 is configured to detect the deformation of the stem 91, and the second pressure sensing circuit 82 is configured to detect the deformation of the support steel sheet 92.

It may be understood that the two pressure sensing circuits, that is, the first pressure sensing circuit 81 and the second pressure sensing circuit 82, are disposed, and the first pressure sensing circuit 81 and the second pressure sensing circuit 82 are spaced apart. In comparison with an implementation in which only one pressure sensing circuit 80 is disposed, in this implementation, a plurality of pressure sensing circuits 80 are disposed, so that pressure sensing detection accuracy is higher. This helps improve collection of the ECG signal and the PPG signal. In addition, when one of the pressure sensing circuits 80 fails, another pressure sensing circuit 80 may still operate normally, and a pressure sensing detection function of the health monitoring module 100 is reliable.

As shown in FIG. 17 to FIG. 19, the circuit board 30, the first pressure sensing circuit 81, the stem 91, the elastic member 93, a part of the support steel sheet 92, and the second pressure sensing circuit 82 are sequentially disposed from top to bottom in the Z-axis direction.

For example, in a pressure detection process in this embodiment, the health monitoring module 100 may move towards the middle frame 220 in the thickness direction of the health monitoring module 100, and the first surface 911 of the stem 91 is deformed based on an action force between the second waterproof member 58 and the middle frame 220. In other words, the stepped face 913 abutting against the middle frame 220 may not need to be disposed on the health monitoring module 100 in this embodiment.

For a specific disposing manner of the stem 91, the second pressure sensing circuit 82, and the elastic member 93, refer to the disposing manner of the stem 91, the pressure sensing circuit 80, and the elastic member 93 in the embodiment shown in FIG. 14. Details are not described herein again.

For example, when the health monitoring module is mounted on the middle frame 220, the first pressure sensing circuit 81 may be located on an outer side of the middle frame 220, and the second pressure sensing circuit 82 may be located on an inner side of the middle frame 220.

It may be understood that embodiments of this application and features in embodiments may be combined with each other if there is no conflict, and any combination of features in different embodiments also falls within the protection scope of this application. In other words, the plurality of embodiments described above may be further combined based on an actual requirement.

It may be understood that all the foregoing accompanying drawings are example illustrations of this application, and do not represent actual sizes of products. In addition, a size proportion relationship between components in the accompanying drawings is not intended to limit an actual product in this application.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A health monitoring module (100), comprising an enclosure (10), an optical heart rate module (20), a circuit board (30), and a light-transmitting member (40), wherein the enclosure (10) is fastened to a side of the circuit board (30), the enclosure (10) and the circuit board (30) jointly enclose accommodation space (50), a light-transmitting hole (11) is disposed in the enclosure (10), and the light-transmitting hole (11) communicates the accommodation space (50) with the outside;
the enclosure (10) comprises a conductive portion (1), the conductive portion (1) is made of a conductive material, a part of the conductive portion (1) protrudes from a top face (12) of the enclosure (10), and another part is electrically connected to the circuit board (30);
the light-transmitting member (40) is fastened to the enclosure (10) and at least partially filled in the light-transmitting hole (11); and
the optical heart rate module (20) is located in the accommodation space (50), the optical heart rate module (20) is fastened to the circuit board (30) and is electrically connected to the circuit board (30), the optical heart rate module (20) and the light-transmitting hole (11) are disposed oppositely, and the optical heart rate module (20) emits and receives light beams through the light-transmitting hole (11) and the light-transmitting member (40).

2. The health monitoring module (100) according to claim 1, wherein the health monitoring module (100) further comprises a pressure sensing circuit (80) and a pressure sensing assembly (90), the pressure sensing assembly (90) is fastened to a side that is of the circuit board (30) and that faces away from the enclosure (10), and the pressure sensing circuit (80) is fastened to the pressure sensing assembly (90); and
when the enclosure (10) is subject to pressure, and the pressure sensing assembly (90) is deformed, the pressure sensing circuit (80) is configured to detect the deformation of the pressure sensing assembly (90).

3. The health monitoring module (100) according to claim 2, wherein the pressure sensing assembly (90) comprises a stem (91), and a part of region of a first surface (911) of the stem (91) is fastened to the side that is of the circuit board (30) and that faces away from the enclosure (10);
the pressure sensing circuit (80) is disposed in another part of region of the first surface (911) of the stem (91), and the pressure sensing circuit (80) and the circuit board (30) are spaced apart; and
when the enclosure (10) is subject to the pressure, and the stem (91) is deformed, the pressure sensing circuit (80) is configured to detect the deformation of the stem (91).

4. The health monitoring module (100) according to claim 3, wherein a groove (9111) is disposed on the first surface (911) of the stem (91), and the pressure sensing circuit (80) is disposed in the groove (9111).

5. The health monitoring module (100) according to claim 3 or 4, wherein the stem (91) comprises a first part (914) and a second part (915), the first part (914) is plate-shaped, the second part (915) is rod-shaped, the second part (915) is located on a side that is of the first part (914) and that faces away from the circuit board (30), and an end of the second part (915) is connected to a middle of the first part (914); and
the first surface (911) of the stem (91) is a surface of a side that is of the first part (914) and that faces away from the second part (915), and when the enclosure (10) is subject to the pressure, and the first part (914) is deformed, the pressure sensing circuit (80) is configured to detect the deformation of the first part (914).

6. The health monitoring module (100) according to claim 5, wherein the health monitoring module (100) further comprises a first waterproof member (59), and the first waterproof member (59) is connected to the first part (914) and covers the pressure sensing circuit (80); and/or
the health monitoring module (100) further comprises a second waterproof member (58), and the second waterproof member (58) is sleeved on the second part (915).

7. The health monitoring module (100) according to claim 2, wherein the pressure sensing assembly (90) comprises a stem (91) and a support steel sheet (92), and a first surface (911) of the stem (91) is fastened to the side that is of the circuit board (30) and that faces away from the enclosure (10);
the support steel sheet (92) is connected to a second surface (912) of the stem (91), the first surface (911) of the stem (91) and the second surface (912) of the stem (91) are disposed back to back, and the pressure sensing circuit (80) is fastened to a side that is of the support steel sheet (92) and that is away from the stem (91); and
when the enclosure (10) is subject to the pressure, and the support steel sheet (92) is deformed, the pressure sensing circuit (80) is configured to detect the deformation of the support steel sheet (92).

8. The health monitoring module (100) according to claim 7, wherein the pressure sensing assembly (90) further comprises an elastic member (93), the elastic member (93) abuts against the stem (91) and the support steel sheet (92), and the elastic member (93) is elastic.

9. The health monitoring module (100) according to claim 2, wherein the pressure sensing assembly (90) comprises a stem (91) and a support steel sheet (92), a part of region of a first surface (911) of the stem (91) is fastened to the side that is of the circuit board (30) and that faces away from the enclosure (10), the support steel sheet (92) is connected to a second surface (912) of the stem (91), and the second surface (912) of the stem (91) and the first surface (911) of the stem (91) are disposed back to back;
the pressure sensing circuit (80) comprises a first pressure sensing circuit (81) and a second pressure sensing circuit (82), the first pressure sensing circuit (81) is disposed in another part of region of the first surface (911) of the stem (91), the first pressure sensing circuit (81) and the circuit board (30) are spaced apart, and the second pressure sensing circuit (82) is fastened to a side that is of the support steel sheet (92) and that is away from the stem (91); and
when the enclosure (10) is subject to the pressure, the stem (91) is deformed, and the support steel sheet (92) is deformed, the first pressure sensing circuit (81) is configured to detect the deformation of the stem (91), and the second pressure sensing circuit (82) is configured to detect the deformation of the support steel sheet (92).

10. The health monitoring module (100) according to any one of claims 1 to 9, wherein the enclosure (10) and the light-transmitting member (40) are an integrally formed mechanical part; and/or
the light-transmitting member (40) is in contact with and fastened to the enclosure (10), the optical heart rate module (20), and the circuit board (30).

11. The health monitoring module (100) according to any one of claims 1 to 9, wherein a first through hole (31) and a second through hole (32) that are spaced apart are disposed on the circuit board (30), both the first through hole (31) and the second through hole (32) communicate the accommodation space (50) with the outside, and the light-transmitting member (40) is filled in the accommodation space (50), the first through hole (31), and the second through hole (32);
the optical heart rate module (20) comprises a light emitter (21) and a light receiver (22), and the light emitter (21) and the light receiver (22) are spaced apart on the circuit board (30); and
the light emitter (21) is located between the first through hole (31) and the second through hole (32), or the light receiver (22) is located between the first through hole (31) and the second through hole (32).

12. The health monitoring module (100) according to any one of claims 1 to 9, wherein the optical heart rate module (20) comprises a light emitter (21) and a light receiver (22), the light emitter (21) and the light receiver (22) are spaced apart and fastened to the circuit board (30), there are two light-transmitting holes (11), the two light-transmitting holes (11) are spaced apart, and the light emitter (21) and the light receiver (22) are respectively disposed in correspondence with the two light-transmitting holes (11); and
the enclosure (10) comprises a frame portion (19) and a light-shielding portion (15), the frame portion (19) is connected to the circuit board (30), the frame portion (19) and the circuit board (30) enclose the accommodation space (50), the light-transmitting holes (11) are disposed in the frame portion (19), the light-shielding portion (15) is connected to the frame portion (19) and located in the accommodation space (50), the light-shielding portion (15) is at least partially located between the light emitter (21) and the light receiver (22), and the light-shielding portion (15) is made of an opaque material.

13. The health monitoring module (100) according to any one of claims 1 to 12, wherein the optical heart rate module (20) and the enclosure (10) are spaced apart.

14. The health monitoring module (100) according to claim 13, wherein the light-transmitting member (40) is further filled in the accommodation space (50) and wraps the optical heart rate module (20).

15. The health monitoring module (100) according to any one of claims 1 to 14, wherein the health monitoring module (100) further comprises a conductive member (60), the conductive member (60) is located in the accommodation space (50) and is spaced apart from the optical heart rate module (20), the conductive member (60) abuts against the conductive portion (1) and the circuit board (30) and is electrically connected between the conductive portion (1) and the circuit board (30), and the conductive member (60) is elastic.

16. The health monitoring module (100) according to claim 15, wherein a hole (63) is disposed on the conductive member (60); and/or
a part of region of a first surface (61) of the conductive member (60) is connected to the circuit board (30), another part of region of the first surface (61) of the conductive member (60) is recessed in a direction away from the circuit board (30) and is spaced apart from the circuit board (30).

17. The health monitoring module (100) according to any one of claims 1 to 16, wherein the health monitoring module (100) further comprises a conducting layer (70) disposed on the top face (12) of the enclosure (10), the conducting layer (70) is electrically connected to the conductive portion (1), and material hardness of the conducting layer (70) is greater than material hardness of the conductive portion (1).

18. The health monitoring module (100) according to any one of claims 1 to 17, wherein the health monitoring module (100) further comprises a flexible printed circuit board (99), the flexible printed circuit board (99) is connected to the circuit board (30) and is electrically connected to the circuit board (30), and the flexible printed circuit board (99) is located outside the accommodation space (50).

19. An electronic device (1000), comprising a housing (200) and the health monitoring module (100) according to any one of claims 1 to 18, wherein the health monitoring module (100) is mounted on the housing (200).

20. The electronic device (1000) according to claim 19, wherein the electronic device (1000) further comprises an ECG chip, and the ECG chip is located inside the housing (200) and is electrically connected to the conductive portion (1) of the enclosure (10) of the health monitoring module (100).

21. The electronic device (1000) according to claim 19 or 20, wherein the electronic device (1000) further comprises a screen (210), the housing (200) comprises a middle frame (220) and a rear cover (230), the middle frame (220) is connected between the screen (210) and the rear cover (230), and the health monitoring module (100) is mounted on the middle frame (220) and protrudes from an outer surface (2201) of the middle frame (220).

22. The electronic device (1000) according to any one of claims 19 to 21, wherein the electronic device (1000) is a watch or a band, and the health monitoring module (100) is a button of the watch or the band.

23. The electronic device (1000) according to any one of claims 19 to 22, wherein the electronic device (1000) further comprises a motor, the motor is mounted on the housing (200), and the motor vibrates based on data monitored by the health monitoring module (100).
